# EUROPEAN PATENT APPLICATION

(11) **EP 4 821 481 A1**
(43) Date of publication of application: **14.10.2026**
(21) Application number: 24900735.2
(22) Date of filing: 12.01.2024
(51) Int. Cl.: A61K 9/00, A61K 47/42, A61K 45/06, A61P 35/00, C12N 9/26

(54) **COMPOSITION FOR SUBCUTANEOUS ADMINISTRATION COMPRISING HYALURONIDASE POLYPEPTIDE AND DRUG**

(30) Priority: 06.12.2023 KR 20230175863
(71) Applicant: BMI Korea Co., Ltd, Jeju-si, Jeju-do 63309 (KR)
(72) Inventor: WOO, Koo, Jeju-si, Jeju-do 63093 (KR); SHIN, Jaewook, Cheongju-si, Chungcheongbuk-do 28464 (KR); KIM, Nayoung, Seoul 08527 (KR)
(74) Representative: Grünecker Patent- und Rechtsanwälte PartG mbB
(86) International application number: PCT/KR2024/000640
(87) International publication number: WO 2025/121541

(57) **Abstract**

Disclosed herein is a composition for subcutaneous injection comprising a hyaluronidase polypeptide and a drug.

## Description

### TECHNICAL FIELD

The present disclosure relates to a composition for subcutaneous injection comprising a hyaluronidase polypeptide and a drug.

### BACKGROUND ART

Hyaluronidases are a generic term for a family of enzymes that catalyze the degradation of hyaluronic acid. At first, Duran-Reynals introduced hyaluronidase as a spreading factor. Later, it has been called hyaluronidase as it was observed to exhibit potent activity on hyaluronic acid. According to the enzymatic mechanism thereof, the enzymes are classified into the following three types: hyaluronate 4-glycanohydrolases (EC 3.2.1.35) distributed in testicles, lysosomes, and bee venoms; hyaluronate 3-glycanohydrolases (EC 3.2.1.36) found in leaches; and hyaluronate lyases (EC 4.2.2.1) found in bacteria.

In particular, hyaluronidase (PH-20) in testicles is a glycosylphosphatidylinositol (GPI)-anchored enzyme present on the acrosomal part of a sperm and is an important enzyme that causes fertilization by degrading the thick outer wall of the egg. In addition, PH-20 is known to cleave the β(1-4) linkage between D-glucuronic acid and N-acetyl-D-glucosamine found in hyaluronic acid, chondroitin, and chondroitin sulfate among glycosaminoglycans distributed in the mammalian skin. The general molecular formula of these enzymes is C₂₄₅₅H₃₇₇₅N₆₁₇O₇₀₄S₂₁ and a molecular weight is 53870.9 g/mol. In humans, there are six genes associated with the enzymes comprising HYAL1, HYAL2, HYAL3, and PH-20/SPAM1.

Since the 1950s, broad applications of hyaluronidases have been comprehensively reviewed. The first application was the subcutaneous injection of parenteral fluids. Besides, the enzymes have found applications in the fields of orthopedic, ophthalmology, plastic surgery, dental, oral surgery, gynecology, and otorhinolaryngology, such as use in infiltration and block anesthesia in order to increase the diffusion of local anesthetics and steroids, dispersion of collected body fluids, e.g., hematoma, prevention of peritoneal adhesion and calculus formation, and treatment of infertility.

Currently available on the market are hyaluronidases that are extracted from ovine or bovine testicles, as exemplified by Vitrase (ISTA Pharmaceuticals, ovine source), and Amphadase (Amphastar Pharmaceuticals, bovine source). They are commercialized by loading a suitable concentration of unprocessed hyaluronidases into vials, followed by lyophilization. The commercial products of animal-derived hyaluronidases leave many problems in application to various fields because foreign proteins contained therein may cause allergic responses and their biological activity decreases due to the decrease of stability with time.

To overcome such problems, research has been conducted on recombinant hyaluronidase. Recombinant proteins can be expressed in various types of cells comprising E. coli, yeasts, insect cells, animal cells, etc. Particularly as for hyaluronidases, their activities are affected by the glycosylation made in the posttranslational modification process. This is because glycans may have influences on the antigenicity, structural folding, solubility, and stability of glycoproteins. From this point of view, animal cells are suitable among various types of expression cells with the most preference for CHO (Chinese hamster ovary) cells which have secured safety, because yeasts or insect cells that glycosylation take place are different from mammalian cells in terms of the posttranslational modification process.

The first recombinant hyaluronidase of PH-20 was developed by Halozyme Therapeutic and has been sold under the tradename of Hylenex, and are being developed for various uses in subcutaneous injection, vitrectomy, and ocular disability, etc. However, hyaluronidases are still poor in yield or stability and are insufficiently supplied compared to demand, so there is a need for a hyaluronidase with improved yield or stability.

### DISCLOSURE

### TECHNICAL PROBLEM

An aspect of the present disclosure is to provide a composition for subcutaneous injection comprising a hyaluronidase polypeptide with excellent stability and improved activity; and a drug.

Another aspect of the present disclosure is to provide a pharmaceutical composition for treating cancer, comprising the hyaluronidase polypeptide according to an aspect of the present disclosure and an anti-cancer agent.

### TECHNICAL SOLUTION

An aspect of the present disclosure pertains to a composition for subcutaneous injection comprising a polypeptide in which a C-terminal region is deleted from the amino acid sequence of wild-type hyaluronidase; and a drug.

Another aspect of the present disclosure pertains to a composition for subcutaneous injection comprising a polypeptide in which the selected number of amino acids from an integer of 1 to 203 are consecutively deleted from the C-terminus of the amino acid sequence of wild-type hyaluronidase; and a drug.

A further aspect of the present disclosure pertains to a pharmaceutical composition for treating cancer, comprising a polypeptide in which a C-terminal region is deleted from the amino acid sequence of wild-type hyaluronidase; and an anti-cancer agent.

A still further aspect of the present disclosure pertains to a pharmaceutical composition for treating cancer comprising a polypeptide in which the selected number of amino acids from an integer of 1 to 203 are consecutively deleted from the C-terminus of the amino acid sequence of wild-type hyaluronidase; and an anti-cancer agent.

Below, a detailed description will be given of the present disclosure.

An aspect of the present disclosure is drawn to a polypeptide having a sequence homology of 90% or higher with a polypeptide in which a C-terminal region is deleted from the amino acid sequence of wild-type hyaluronidase. The wild-type hyaluronidase may be a polypeptide consisting of the amino acid sequence of SEQ ID NO: 1.

The polypeptide according to an aspect of the present disclosure has hyaluronidase activity, and has at least one of the following characteristics (1) to (6):
(1) stable at a pH range of pH 3 to 10, pH 3 to 9, pH 3 to 8, pH 3 to 7, pH 3 to 6.5, pH 3 to 6, pH 3 to 5.5, pH 3 to 5, pH 3 to 4.5, pH 4 to 10, pH 4 to 9, pH 4 to 8, pH 4 to 7, pH 4 to 6.5, pH 4 to 6, pH 4 to 5.5, pH 4 to 5, pH 4 to 4.5, pH 4.5 to 10, pH 4.5 to 9, pH 4.5 to 8, pH 4.5 to 7, pH 4.5 to 6.5, pH 4.5 to 6, pH 4.5 to 5.5, pH 4.5 to 5, pH 5 to 10, pH 5 to 9, pH 5 to 8, pH 5 to 7, pH 5 to 6.5, pH 5 to 6, or pH 5 to 5.5, specifically, enzyme activity is 57% or higher of the initial activity after storage for 4 weeks at a pH in the range between higher than 3 and 10 or less (for example, more than pH 3 and less than pH 7, more than pH 3 and pH 6.5 or less, more than pH 3 and pH 6 or less, more than pH 3 and pH 5.5 or less, more than pH 3 and pH 5 or less, as one example, pH 5.0, pH 7.0, or pH 10), or enzyme activity is 32% or higher of the initial activity after storage for 4 weeks at a pH of 3 to less than 5 (for example, pH 3 to 4.5, or pH 3 to 4, as one example, pH 3.0),
(2) stable at a temperature of -20 to 45°C, specifically, enzyme activity is 63% or higher of the initial activity after storage at a temperature less than 0°C for 4 weeks, enzyme activity is 83% or higher of the initial activity after storage at a temperature of 0 to 40°C for 4 weeks, or enzyme activity is 52% or higher of the initial activity after storage at a temperature of 40°C or higher for 4 weeks,
(3) high hyaluronidase activity, specifically, hyaluronidase activity is more than 1- to 3-fold compared to wild-type hyaluronidase,
(4) having a titer more than 1- to 3-fold of a titer of polypeptide consisting of the amino acid sequence of SEQ ID NO: 1,
(5) having an activity of 120,000 to 150,000 IU/mg, and
(6) one or more amino acid residue is glycosylated.

In detail, a polypeptide is prepared by deleting a C-terminal region of the amino acid sequence of ovine-derived wild-type hyaluronidase (CAS no.488712-31-8) in the present Example. The polypeptide thus obtained were observed to have stability and activity remarkably higher than those of the wild-type hyaluronidase, and even when used in a smaller amount, exhibited equivalent effects of drug absorption, drug diffusion promoting effects, and body fluid reabsorption effects to those of the wild-type. Therefore, the polypeptide according to an embodiment of the present disclosure may be a hyaluronidase.

In detail, the polypeptide according to an embodiment of the present disclosure may have a sequence homology of 90% or more, 91% or more, 92% or more, 93% or more, 94% or more, 95% or more, 96% or more, 97% or more, 98% or more, 99% or more, 99.5% or more, or 99.9% or more with a polypeptide in which the selected number of amino acids from an integer of 1 to 203 are consecutively deleted from the C-terminus of the amino acid sequence of wild-type hyaluronidase. In this regard, the polypeptide according to an embodiment of the present disclosure is not the polypeptide consisting of the amino acid sequence of SEQ ID NO: 1. In addition, the polypeptide according to an embodiment of the present disclosure may retain the catalytic activity of the wild-type hyaluronidase. Specifically, the polypeptide according to an embodiment of the present disclosure may have activity and/or stability equal to or greater than that of wild-type hyaluronidase. The wild-type hyaluronidase may be a polypeptide consisting of the amino acid sequence of SEQ ID NO: 1.

Specifically, the polypeptide according to an embodiment of the present disclosure may be a C-terminal region deletion variant in which n amino acids from the C-terminus of the amino acid sequence of wild-type hyaluronidase is consecutively deleted (wherein, n is a natural number of 1 to 203).

By way of example, the polypeptide according to an embodiment of the present disclosure may have consecutive deletion of the number selected from an integer of 1 to 203, the number selected from an integer of 1 to 170, the number selected from an integer of 1 to 136, the number selected from an integer of 1 to 102, the number selected from an integer of 1 to 68, the number selected from an integer of 34 to 203, the number selected from an integer of 34 to 170, the number selected from an integer of 34 to 136, the number selected from an integer of 34 to 102, the number selected from an integer of 34 to 68, the number selected from an integer of 68 to 203, the number selected from an integer of 68 to 170, the number selected from an integer of 68 to 136, the number selected from an integer of 68 to 102, 34, 68, 102, 136, or 170 amino acids from the C-terminus of the amino acid sequence of wild-type hyaluronidase. The polypeptide according to an embodiment of the present disclosure may further have deletion of the first amino acid from the N-terminus of the amino acid sequence of wild-type hyaluronidase. The amino acid sequence of the wild-type hyaluronidase may be the amino acid sequence of SEQ ID NO: 1.

For one example, the polypeptide according to an embodiment of the present disclosure may consist of an amino acid sequence extending from the first amino acid to the m^{th} amino acid or the second amino acid to the m^{th} amino acid from the N-terminus of the amino acid sequence of wild-type hyaluronidase (wherein, m is a natural number of 315 to 517). The amino acid sequence of the wild-type hyaluronidase may be the amino acid sequence of SEQ ID NO: 1.

For one example, the polypeptide according to an embodiment of the present disclosure may consist of the amino acid sequence of SEQ ID NO: 2, SEQ ID NO: 3, SEQ ID NO: 4, SEQ ID NO: 5, or SEQ ID NO: 6.

For one example, the polypeptide according to an embodiment of the present disclosure may be expressed using animal cells as a host. In this regard, the polypeptide according to an embodiment of the present disclosure may be expressed in animal cells as a host, and glycosylated through post-translational modification (PTM) process when being expressed.

The polypeptide according to an embodiment of the present disclosure may be stable at a pH 3 to 10. Specifically, the polypeptide according to an embodiment of the present disclosure may decrease in enzymatic activity upon storage at a pH of 3 to 10 to a lesser extent, compared to the wild-type hyaluronidase.

In the Example of the present disclosure, the polypeptide according to an embodiment of the present disclosure was observed to retain the enzymatic activity after four weeks of storage at a pH of 3 to 10.

For example, the polypeptide according to an embodiment of the present disclosure may have an enzymatic activity amounting to 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 51% or more, 52% or more, 53% or more, 54% or more, or 55% or more of the initial activity thereof after storage at a pH in the range between pH 3 and less than pH 5, or pH 3 to 4, e.g., pH 3 for 4 weeks. In this context, the polypeptide may be stored at a temperature of 5°C or 37°C.

For example, the polypeptide according to an embodiment of the present disclosure may have an enzymatic activity amounting to 57% or more, 58% or more, 59% or more, 60% or more, 61% or more, 62% or more, 63% or more, 64% or more, or 65% or more of the initial activity thereof after storage at a pH in the range between higher than pH 3 and pH 10 or less, pH 4 to 10, or pH 5 to 10, for example, pH 5, pH 7, or pH 10 for 4 weeks. In this context, the polypeptide may be stored at a temperature of 5°C or 37°C.

For example, the polypeptide according to an embodiment of the present disclosure may have an enzymatic activity of 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, or 82% or more of the initial activity thereof after storage at a pH in the range between higher than pH 3 and less than pH 10, between higher than pH 3 and pH 9 or less, between higher than pH 3 and pH 8 or less, between higher than pH 3 and pH 7 or less, between pH 4 or more and less than pH 10, pH 4 to 9, pH 4 to 8, pH 4 to 7, between pH 5 and less than pH 10, pH 5 to 9, pH 5 to 8, or pH 5 to 7, e.g., pH 5 or pH 7 for 4 weeks. In this context, the polypeptide may be stored at a temperature of 5°C or 37°C.

Specifically, the polypeptide according to an example of the present disclosure may be stable under slightly acidic conditions, for example, pH 3 to 6.5, pH 3 to 6, pH 3 to 5.5, pH 3 to 5, pH 3 to 4.5, pH 4 to 4.5, pH 4 to 5, pH 4 to 5.5, pH 4 to 6, pH 4 to 6.5, pH 4.5 to 5, pH 4.5 to 5.5, pH 4.5 to 6, pH 4.5 to 6.5, pH 5 to 5.5, pH 5 to 6, or pH 5 to 6.5. pH is an important factor in the stability of drugs, and especially for antibody drugs, it ranges from pH 5 to 6.5 or pH 5 to 6. Conventional hyaluronidase is stable at neutral pH, so when conventional hyaluronidase and antibody drugs are mixed and stored for a long time, stability due to pH may be problematic. The polypeptide according to an example of the present disclosure is stable at the slightly acidic conditions similar to the pH of the antibody drugs (e.g., pH 5.0), and is advantageous for preparation as a mixed formulation with the antibody drugs, and advantageous for formulation and long-term storage.

The polypeptide according to an embodiment of the present disclosure may be stable in a frozen, a refrigerated, and a high-temperature condition. In detail, when stored in a frozen, a refrigerated, and a high-temperature condition, the polypeptide according to an embodiment of the present disclosure decreases in enzymatic activity to a lesser extent, compared to the wild-type hyaluronidase.

In the present Example, the polypeptide according to an embodiment of the present disclosure was observed to retain an enzymatic activity after 4 weeks of storage in a frozen (e.g., -18°C to -20°C), a refrigerated (e.g., 2 to 8°C), and a high-temperature (e.g., 40 to 45°C) condition.

For example, the polypeptide according to an embodiment of the present disclosure may have an enzymatic activity amounting to 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more of the initial activity after storage at a frozen temperature, for example, a temperature of less than 0°C, -20 to less than 0°C, -20 to -10°C, or -20 to - 18°C, for one example, at a temperature of -20°C, for 4 weeks. In this regard, the polypeptide may be stored at pH 5 or 7.

For example, the polypeptide according to an embodiment of the present disclosure may have an enzymatic activity amounting to 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more of the initial activity after storage at a refrigerated temperature, for example, a temperature of 0 to 40°C, 0 to 10°C, or 2 to 8°C, e.g., 5°C for 4 weeks. In this regard, the polypeptide may be stored at pH 5 or 7.

For example, the polypeptide according to an embodiment of the present disclosure may have an enzymatic activity amounting to 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, or 79% or more of the initial activity after storage at a high temperature, for example, a temperature of 40°C or more, 40 to 50°C, or 40 to 45°C, e.g., 40°C for 4 weeks. In this regard, the polypeptide may be stored at a pH of 5 or 7.

The polypeptide according to an embodiment of the present disclosure may have a titer higher than that of the wild-type hyaluronidase. For example, the polypeptide according to an embodiment of the present disclosure may have a titer more than 1- to 3-fold, more than 1- to 2.5-fold, more than 1- to 2-fold, more than 1- to 1.9-fold, more than 1- to 1.8-fold, more than 1- to 1.7-fold, 1.1- to 3-fold, 1.1- to 2.5-fold, 1.1- to 2-fold, 1.1- to 1.9-fold, 1.1- to 1.8-fold, 1.1- to 1.7-fold, 1.2- to 3-fold , 1.2- to 2.5-fold , 1.2-to 2-fold, 1.2- to 1.9-fold, 1.2- to 1.8-fold, 1.2- to 1.7-fold, 1.3- to 3-fold, 1.3- to 2.5-fold, 1.3- to 2-fold, 1.3- to 1.9-fold, 1.3- to 1.8-fold, 1.3- to 1.7-fold, 1.4- to 3-fold, 1.4- to 2.5-fold, 1.4- to 2-fold, 1.4- to 1.9-fold, 1.4- to 1.8-fold, 1.4- to 1.7-fold, 1.5- to 3-fold, 1.5- to 2.5-fold, 1.5- to 2-fold, 1.5- to 1.9-fold, 1.5- to 1.8-fold, or 1.5- to 1.7-fold of a titer of the wild-type hyaluronidase.

For example, the polypeptide according to an embodiment of the present disclosure may have an activity of 120,000 to 150,000 IU/mg, 120,000 to 145,000 IU/mg, 120,000 to 140,000 IU/mg, 120,000 to 135,000 IU/mg, 121,000 to 150,000 IU/mg, 121,000 to 145,000 IU/mg, 121,000 to 140,000 IU/mg, 121,000 to 135,000 IU/mg, 122,000 to 150,000 IU/mg, 122,000 to 145,000 IU/mg, 122,000 to 140,000 IU/mg, or 122,000 to 135,000 IU/mg.

In the Example of the present disclosure, when topically administered, the polypeptide according to an embodiment of the present disclosure was observed to exhibit remarkably better effects of drug absorption and diffusion promoting, compared to the wild-type hyaluronidase.

In addition, in the present Example, when topically administered, the polypeptide according to an embodiment of the present disclosure was observed to exhibit a remarkably higher effect of promoting reabsorption of excessive body fluid, compared to the wild-type hyaluronidase. Thus, the polypeptide according to an embodiment of the present disclosure can be used in combination with a drug for subcutaneous administration of the drug.

A drug comprised in the composition according to an embodiment of the present disclosure comprises a protein medicine, antibody, an aptamer, mRNA, siRNA (small interfering RNA), shRNA (short hairpin RNA), miRNA (micro RNA), aiRNA (asymmetric interfering RNA), RNAi (RNA interference), antisense oligonucleotide, or small molecule.

The drug may be a soluble receptor, or a soluble receptor and Fc fusion protein.

The drug may be an anti-cancer agent, anti-diabetic agent, anti-inflammatory agent, anti-viral agent, or immunomodulatory agent. The immunomodulatory agent may be, for example, an autoimmune disease treating agent.

The protein medicine is a drug that is composed of amino acids and shows a disease treatment or prevention effect through the activity of the protein, refers to a medicine made up of proteins other than antibody medicines, for example, it may comprise one or more selected from the group consisting of cytokines, therapeutic enzymes, hormone, soluble receptor and its fusion protein, insulin or its analogue, BMP (Bone Morphogenetic Protein), EPO (erythropoietin), and plasma-derived protein (serum derived protein).

The cytokine may comprise one or more selected from the group consisting of interferon, interleukin, CSF (colony stimulating factor), TNF (tumor necrosis factor), and TGF (tissue growth factor).

The cytokine and its similar products include Oprelvekin, Peginterferon Beta-1a, Pegilodecakin, Rezpegaldesleukin, SAR 444245, GO-203-2c, Dekavil, Nemvaleukin alfa, Efavaleukin alfa, Efineptakin alfa, Onfekafusp alfa, Bifikafusp alfa, or Lerodalcibep and the like, but is not limited thereto.

The therapeutic enzyme may comprise one or more selected from the group consisting of β-glucocerebrosidase and agalsidase β.

The soluble receptor refers to an extracellular domain of the receptor, and its fusion protein refers to a fused protein in which the Fc region of an antibody and the like is fused to the soluble receptor. The soluble receptor is a water-soluble form of the receptor to which a disease-related ligand binds, and a form in which the Fc region is fused to a TNF-α soluble receptor (e.g., a product with the ingredient name Etanercept and similar forms), a from in which the Fc region is fused to VEGF soluble receptors (e.g., products with the ingredient names Aflibercept, Conbercept, or OPT-302 and similar forms), a form in which the Fc region is fused to CTLA-4 (e.g., products with the ingredient names Abatacept or Belatacept and similar forms), a form in which the Fc region is fused to an interleukin soluble receptor (e.g., products with the ingredient names Rilonacept or Invakicept and similar forms), a form in which the Fc region is fused to the LFA3 soluble receptor (e.g., a product with the ingredient name Alefacept and similar forms), a form in which the Fc region is fused to signal regulatory protein alpha (SIRPalpha) that interacts with CD47 (e.g., products with the ingredient names Evorpacept, Ontorpacept, or TTI-622, and similar forms), products with the ingredient names Sotatercept, Dalantercept, Acazicolcept, or Dazodalibep and similar forms, and the like may be exemplified, but is not limited thereto.

The hormone refers to a hormone or its analogue injected from outside the body for the treatment or prevention of diseases caused by hormone deficiency and the like, and human growth hormone, estrogen, and progesterone and the like may be exemplified, but is not limited thereto.

The plasma derived protein refers to proteins present in plasma, both those extracted from plasma and those produced recombinantly, and fibrinogen, von Willebrand Factor, albumin, thrombin, FII (Factor II), FV (Factor V), FVII (Factor VII), FVIII (Factor VIII), FIX (Factor IX), FX (Factor X), and FXI (Factor XI) may be exemplified, but is not limited thereto.

The antibody medicine may be a monoclonal antibody drug or a polyclonal antibody drug. An antibody may be a complete antibody or a binding fragment thereof, and the "antigen-binding fragment" is a fragment of the entire immunoglobulin structure and refers to a portion of a polypeptide containing a part to which an antigen can bind. For example, it may be scFv, (scFv)2, scFvFc, Fab, Fab' or F(ab')2, but is not limited thereto. The antigen-binding fragment of the antibody in the present disclosure is an antibody fragment comprising one or more of the complementary determining regions, for example, may be selected from the group consisting of scFv, (scFv)2, scFv-Fc, Fab, Fab', and F(ab')2. The antigen-binding fragment can be obtained using a protease (for example, Fab can be obtained when the entire antibody is restriction cleaved with papain, and F(ab')2 fragment can be obtained when cleaved with pepsin), and can be produced through genetic recombination technology.

The antibody or antigen-binding fragment may be isolated from a living body or may be non-naturally occurring. The antibody or antigen-binding fragment may be produced synthetically or recombinantly.

The monoclonal antibody drug refers to a monoclonal antibody that can specifically bind to an antigen related to a specific disease and a protein containing a fragment of a monoclonal antibody. A monoclonal antibody also includes a bispecific/multispecific antibody, and a monoclonal antibody or a protein containing a fragment thereof is used to include antibody-drug conjugate (ADC).

For example, an antigen related to a specific disease may be exemplified as 4-1BB, 5T4, integrin, Activin, amyloid beta, angiopoietin (angiopoietin 1 or 2), and angiopoietin-like 3, B cell maturation antigen (BCMA), B-cell activating factor (BAFF), B7-H3, complement 5, CCR4, CCR5, CCL11, CD2, CD3, CD4, CD6, CD11a, CD16A, CD19, CD20, CD22, CD25, CD27, CD28, CD30, CD32B, CD33, CD38, CD40, CD45, CD46, CD47, CD52, CD56, CD62, CD70, CD73, CD74, CD79b, CD80, CD105, CD123, CD154, CD166, CD262, CD278, CD319, CD326, Carcinoembryonic antigen (CEA), CGRP, Claudin-18, c-Met, CSF-1, CSF-1 Receptor, CTLA4, DLL3, EGF receptor, hemophilia factor, Fc receptor, FGF23, folate receptor, GD2, Glucocorticoid-induced TNF receptor (GITR), Glypican 3, GM-CSF, HER2, HER3, Hepatocyte Growth Factor (HGF), interferon receptor, interferon gamma, IgE, IGF-1 receptor, interleukin 1, interleukin 2 receptor, interleukin 4, interleukin 4 receptor, interleukin 5, interleukin 5 receptor, interleukin 6, interleukin 6 receptor, interleukin 8, interleukin 12/23, interleukin 13, interleukin 17A, interleukin 17 receptor A, interleukin 23, interleukin 31 receptor, interleukin 36 receptor, Lymphocyte-activation gene 3 (LAG3), Lysyl Oxidase Homolog 2 (LOXL2), Mesothelin, Mucin-1, Mucin-16, Netin-4, Nerve Growth Factor (NGF), OX40, Proprotein Convertase Subtilisin/Kexin type 9 (PCSK9), PD-1, PD-L1, Phospholipase C, RANKL (Receptor activator of nuclear factors kappa Bligand), Tyrosine-protein kinase transmembrane receptor (ROR1), Sialic acid binding ig-like lectin 15 (Siglec-15), Transforming Growth Factor beta (TGFβ), TIGIT (T-cell immunoreceptor with immunoglobulin and ITIM domain), T cell immunoglobulin and mucin-domain containing-3 (Tim-3), Tissue factor, Tissue factor pathway inhibitor (TFPI), TORP-2, tumor necrosis factor (TNF), Thymic stromal lymphopoietin (TSLB), Colony stimulating factor 1 receptor (CSF1R), Vascular endothelial growth factor (VEGF), VEGF receptor, and vWF (von Willebrand Factor) and the like, but not limited thereto.

The monoclonal antibody and the protein containing a monoclonal antibody fragment may be exemplified as ADG-106, EU-101, LVGN6051, Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab pegol, Iratumumab, BI-1206, Lintuzumab, Daratumumab, Felzartamab, GEN3014, Isatuximab, Mezagitamab, CDX-1140, Bleselumab, Dacetuzumab, Iscalimab, Lucatumumab, Mitazalimab, SEA-CD40, Sotigalimab, SAR441344, Tegoprubart, Dapirolizumab pegol, I-131-Apamistamab, AO-176, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, TST001, ZL-1211, Zolbetuximab, Onartuzumab, Eculizumab, Pozelimab, Ravulizumab, Lacnotuzumab, PD-0360324, AMB-051, Axatilimab, Cabiralizumab, Emactuzumab, BA3071, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, MK-2060, Batoclimab, Nipocalimab, Rozanolixizumab, Burosumab, Farletuzumab, Dinutuximab, Dinutuximab beta, Naxitamab, BMS-986156, Ragifilimab, Gimsilumab, Lenzilumab, Mavrilimumab, Namilumab, Otilimab, Plonmarlimab, Codrituzumab, Margetuximab, Pertuzumab, Trastuzumab, HMBD-001, Patritumab, Seribantumab, Duligotuzumab, Ficlatuzumab, Rilotumumab, Alomfilimab, Anifrolumab, Emapalumab, FB825, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, CBP201, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, HuMax-IL8, Abituzumab, Favezelimab, Fianlimab, GSK2831781, Ieramilimab, INCAGN02385, Relatlimab, Simtuzumab, Abagovomab, Oregovomab, Tanezumab, BMS-986178, GSK3174998, INCAGN01949, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, MEDI0680, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, IMC-001, Envafolimab, Suvratoxumab, Denosumab, Zilovertamab, NC318, Elotuzumab, NIS793, BMS-986207, Domvanalimab, EOS-448, Etigilimab, Ociperlimab, Tiragolumab, Vibostolimab, Surzebiclimab, Cobolimab, Sabatolimab, TQB2618, Concizumab, Marstacimab, Adalimumab, Golimumab, Infliximab, Certolizumab pegol, Conatumumab, Tigatuzumab, Tezepelumab, Gatipotuzumab, Cabiralizumab, Bevacizumab, Brolucizumab , Ranibizumab, Olinvacimab, Icrucumab, Ramucirumab, Caplacizumab, Abrilumab, Etrolizumab, Vedolizumab, Intetumumab, Natalizumab, and the like, but not limited thereto.

The bispecific/multispecific antibody and antibody-like protein include DSP107, RO7122290, Cinrebafusp alfa, GEN1042, Rozibafusp alfa, GEN1044, Obrindatamab, GEN1047, Elranatamab, Linvoseltamab, Teclistamab, Epcoritamab, Glofitamab, Mosunetuzumab, Odronextamab, Flotetuzumab, Vibecotamab, Catumaxomab, Cibisatamab, TAK-186, Talquetamab, Ubamatamab, NVG-111, Emfizatamab, HPN536, AFM13, Blinatumomab, ISB1442, CPO107, AFM24, Amivantamab, MCLA-129, SI-B001, Emicizumab, Mim8, Zenocutuzumab, Zanidatamab, Tibulizumab, GI-101, Vobarilizumab, REGN5668, HX009, Cadonilimab, Vudalimab, EMB-02, RO7247669, Tebotelimab, IBI318, AZD2936, AZD7789, RO7121661, Ivonescimab, IBI322, ES101, GEN1046, PRS-344, Erfonrilimab, FS118, Bintrafusp alfa, HB0036, HLX301, NM21-1480, Ozoralizumab, BI836880, Faricimab, Vanucizumab, Navicixizumab, IBI302, and the like, but not limited thereto.

The antibody-drug conjugates include Naptumomab estafenatox, Belantamab mafodotin, DS-7300, MGC018, Pivekimab sunirine, Praluzatamab ravtansine, Coltuximab ravtansine, Denintuzumab mafodotin, Loncastuximab tesirine, Ibritumomab tiuxetan, Inotuzumab ozogamicin, Epratuzumab-cys-tesirine, Moxetumomab pasudotox, Brentuximab vedotin, Gemtuzumab ozogamicin, Vadastuximab talirine, STI-6129, FOR46, Lorvotuzumab mertansine, Polatuzumab vedotin, Tusamitamab ravtansine, Telisotuzumab vedotin, Rovalpituzumab tesirine, Depatuxizumab mafodotin, Farletuzumab ecteribulin , Mirvetuximab soravtansine, ARX788, Trastuzumab deruxtecan, Trastuzumab duocarmazine, A166, Trastuzumab emtansine, DP303c , MRG002, BDC-1001, SHR-A1811, Disitamab vedotin, Pertuzumab zuvotolimod, Patritumab Deruxtecan, Anetumab ravtansine, BMS-986148, Enfortumab vedotin, NBE-002, MRG004, ABBV-3373, Sacituzumab Govitecan, and the like, but not limited thereto.

The polyclonal antibody may be a serum antibody extracted from plasma such as immune globulin, but not limited thereto.

The composition for subcutaneous injection according to an embodiment of the present application may further comprise a stabilizing agent. The stabilizing agent may comprise one or more selected from the group consisting of buffer, a nonionic surfactant, a chelating agent, alkaline metal salt, and alkaline earth metal salt.

The buffer may be one or more selected from the group consisting of succinate buffer solution, acetate buffer solution, phosphate buffer solution, citrate buffer solution, malonate buffer solution, MES (2-(N-morpholino)ethanesulphonic acid) buffer solution, Tris buffer solution, and glycine buffer solution.

The nonionic surfactant may be polyoxyethylene-sorbitan fatty acid ester.

The nonionic surfactant may be one or more selected from the group consisting of Polysorbate 20, Polysorbate 80, and Triton X-100.

The chelating agent may be EDTA.

The alkaline earth metal salt may be MgCl₂ and/or MgSO₄.

Another embodiment of the present disclosure is drawn to a pharmaceutical composition for treatment of cancer, comprising the hyaluronidase polypeptide according to an embodiment of the present disclosure and an anti-cancer agent.

The cancer is not particularly limited and comprises both solid cancer and hematological cancer. For example, it may be one or more selected from the group consisting of skin cancer such as melanoma, liver cancer, hepatocellular carcinoma, stomach cancer, breast cancer, lung cancer, ovarian cancer, bronchial cancer, nasopharyngeal cancer, laryngeal cancer, pancreatic cancer, bladder cancer, colorectal cancer, colon cancer, uterine cervical cancer, brain cancer, prostate cancer, bone cancer, thyroid cancer, parathyroid cancer, kidney cancer, esophageal cancer, biliary tract cancer, testis cancer, rectal cancer, head and neck cancer, cervical cancer, ureteral cancer, osteosarcoma, neuroblastoma, fibrosarcoma, rhabdomyosarcoma, astrocytoma, neuroblastoma and glioma, but not limited thereto.

The composition according to an embodiment of the present disclosure may further comprise at least one active ingredient that exhibits an equivalent or similar function.

In addition, according to the conventional techniques known to those skilled in the art, the composition according to an embodiment of the present disclosure, for example, a composition for subcutaneous injection or a pharmaceutical composition, may be formulated with a pharmaceutically acceptable carrier and prepared into a single-dose form or enclosed in a multiple-dose container. As used herein, the term "carrier" refers to a substance that facilitates the incorporation of a matter of interest into cells or tissues. Herein, the term "pharmaceutically acceptable" means pertaining to being physiologically acceptable and not causing gastrointestinal disorders, allergic reactions such as dizziness, or similar reactions when administered to a human.

Any pharmaceutical acceptable carrier may be used so long as it is typically available for formulation. Examples of the pharmaceutical acceptable carrier comprise, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

In addition to the above ingredient, the composition according to the present disclosure, for example, a composition for subcutaneous injection or a pharmaceutical composition may further comprise an additive such as a filler, an antiaggregant, a lubricant, a humectant, a flavor, an emulsifier, a preservative, etc. In the present disclosure, the content of the additive in the composition is not particularly limited, but may be appropriately adjusted within the content range acceptable for typical formulation.

The term "excipient", as used herein, means any substance, not itself a therapeutic agent, used as a carrier or vehicle for delivery of a therapeutic agent to a subject or added to a pharmaceutical composition to improve its handling or storage properties or to permit and facilitate of a unit dose of the composition.

The composition according to the present disclosure, for example, a composition for subcutaneous injection or a pharmaceutical composition, may be formulated into various forms, such as sterile injections, etc., according to purposes and may be administered via various routes, such as topical, subcutaneous, intramuscular injection, etc.

A preferable dosage of the composition according to the present disclosure, for example, a composition for subcutaneous injection or a pharmaceutical composition, may vary in a range thereof depending on a patient's state, weight, age, sex, health condition, dietary constitutional specificity, property of a preparation, a degree of disease, an administration time of the composition, an administration method, an administration period or interval, an excretion rate, and drug form, but may be appropriately selected by those skilled in the art.

The term "effective dosage of the pharmaceutical composition", as used herein, means an amount of the composition comprising an active ingredient sufficient to treat a specific symptom. This may vary depending on a method for formulating the pharmaceutical composition, an administration mode, an administration time and/or an administration route, etc., and may be diversified according to various factors comprising a type and degree of reaction to be achieved by administration of the pharmaceutical composition, a type of an individual for administration, the individual's age, weight, general health condition, disease symptom or severity, sex, diet, excretion, ingredients of other drug compositions to be used for the corresponding individual at the same time or different times, etc., as well as other similar factors well known in a pharmaceutical field, and those skilled in the art may easily determine and prescribe an effective dosage for intended treatment.

The pharmaceutical composition according to the present disclosure may be administered once a day or divided into several times. The pharmaceutical composition of the present invention may be administered as an individual therapeutic agent or in combination with other therapeutic agents, and may be administered sequentially or simultaneously with a conventional therapeutic agent. Considering all the above factors, the pharmaceutical composition of the present disclosure may be administered in such an amount that a maximum effect may be achieved by a minimum amount without a side effect.

Compared to the wild-type hyaluronidase, the polypeptide or the composition according to an embodiment of the present disclosure has a higher titer and thus can achieve an equivalent effect at a lower dose. For example, the polypeptide or the composition according to an embodiment of the present disclosure may be administered to at a daily dose of 90% or less, 85% or less, 80% or less, 75% or less, 70% or less, or 65% or less of that of the wild-type hyaluronidase. The dose may be based on weight or % by weight. In addition, a total daily dose may be divided and administered continuously or discontinuously as necessary.

### ADVANTAGEIOUS EFFECTS

With an improvement in expression level and stability in animal cells, the hyaluronidase polypeptide according to an embodiment of the present disclosure exhibits enzymatic activity equal to or greater than the mature wild-type PH-20. Therefore, the hyaluronidase polypeptide according to an embodiment of the present disclosure has an elevated expression level when expressed in CHO cells compared to the mature wild-type PH-20, and is highly stable, there is an effect of increasing the industrial applicability of various uses.

In addition, with ability to degrade hyaluronic acid as a constituent of the intercellular space, hyaluronidases adjust tissue adhesion to facilitate the penetration and diffusion of drugs, and have the effect of promoting reabsorption of body fluids excessively present in tissues, so that their coverage and application fields have been gradually expanded. However, animal-derived wild-type PH-20 is highly prone to infection with the substance originating from the animal source. The hyaluronidase polypeptide according to an embodiment of the present disclosure is unlikely to cause infection and thus safe, the peptide exhibits high activity at the same dose compared to animal-derived wild-type PH-20, and can further increase industrial applicability for various uses.

### DESCRIPTION OF DRAWINGS

FIGs. 1a to FIG. 1c are drawings of SDS-PAGE showing the pH stability of a polypeptide according to an embodiment of the present disclosure in comparison with wild-type hyaluronidase (FIG. 1a: week 0 of storage, FIG. 1b: week 2 of storage, FIG. 1c: week 4 of storage).
FIGs. 2a to FIG. 2c are drawings of SDS-PAGE showing the temperature stability of a polypeptide according to an embodiment of the present disclosure in comparison with wild-type hyaluronidase (FIG. 2a: week 0 of storage, FIG. 2b: week 2 of storage, FIG. 2c: week 4 of storage; frozen: frozen, refrig.: refrigerated, high tem.: high-temperature).
FIG. 3 shows the results of plate titer assays under various pH and temperature conditions to examine whether a hyaluronic acid solution exhibits its own activity.
FIG. 4a shows the results of titer assays for wild-type hyaluronidase under the temperature condition of 20 to 60°C.
FIG. 4b shows the results of titer assays for a polypeptide according to an embodiment of the present disclosure under the temperature condition of 20 to 60°C.
FIG. 5a shows the results of titer assays for wild-type hyaluronidase under the temperature condition of 35 to 40°C.
FIG. 5b shows the results of titer assays for a polypeptide according to an embodiment of the present disclosure under the temperature condition of 35 to 40°C.
FIG. 6a shows the results of titer assays for wild-type hyaluronidase under the condition of pH 5 to 7.
FIG. 6b shows the results of titer assays for a polypeptide according to an embodiment of the present disclosure under the condition of pH 5 to 7.
FIG. 7 shows plots of blood levels of a polypeptide according to an embodiment of the present disclosure and Hirax against time in SD rats.
FIG. 8 shows the bioavailability of a composition for subcutaneous injection comprising a polypeptide according to an embodiment of the present disclosure and a drug.

### MODE FOR INVENTION

A better understanding of the present disclosure may be obtained in light of following examples which are set forth to illustrate, but are not to be construed to limit, the present disclosure.

### EXAMPLE 1. Preparation of Hyaluronidase

A hyaluronidase was prepared by truncating regions extending from the C-terminus of the amino acid sequence of SEQ ID NO: 1 for the ovine-derived wild-type hyaluronidase (CAS no.488712-31-8) by 34 amino acid residues (Example 1-2), 68 amino acid residues (Example 1-3), 102 amino acid residues (Example 1-4), 136 amino acid residues (Example 1-5), 170 amino acid residues (Example 1-6), or 204 amino acid residues (Example 1-7).

First, a cDNA was synthesized on the basis of the amino acid sequence of wild-type hyaluronidase (CAS no.488712-31-8). The gene of the hyaluronidase was amplified using polymerase chain reaction (hereinafter referred to as "PCR") and examined for expression and activity in CHO-DG44 cells with the aid of pcDNA 3.1 vector. The gene was anchored in CHO-DG44 cells through a pOptiVEC vector. When CHO-DG44 cells reached to a density of 4~6 x 10⁶ cells/mL, the cells were transformed with a plasmid constructed by inserting the hyaluronidase cDNA into pOptiVEC vector, using electroporation. After transformation, the CHO-DG44 cells were cultured in Power CHO 2CD medium (with L-glutamine 4mM) and the cell culture was centrifuged at 12,000 rpm for 10 minutes. The supernatant was recovered and then purified, as necessary, by various methods including affinity chromatography, hydrophobic chromatography, ion exchange chromatography, and the like. The purified hyaluronidase was filtered by replacement with water in ultrafiltration and microfiltration. Amino acid sequences of the hyaluronidases thus obtained are given in Table 1.

**TABLE 1**

| name | Sequence (N → C) | SEQ ID NO: |
|---|---|---|
| Example 1-1 (CAS no.488712-31-8) | LDFRAPPLIS NTSFLWAWNA PAERCVKIFK LPPDLRLFSV KGSPQKSATG QFITLFYADR LGYYPHIDEK TGNTVYGGIP QLGNLKNHLE KAKKDIAYYI PNDSVGLAVI DWENWRPTWA RNWKPKDVYR DESVELVLQK NPQLSFPEAS KIAKVDFETA GKSFMQETLK LGKLLRPNHL WGYYLFPDCY NHNYNQPTYN GNCSDLEKRR NDDLDWLWKE STALFPSVYL NIKLKSTPKA AFYVRNRVQE AIRLSKIASV ESPLPVFVYH RPVFTDGSST YLSQGDLVNS VGEIVALGAS GIIMWGSLNL SLTMQSCMNL GNYLNTTLNP YIINVTLAAK MCSQVLCHDE GVCTRKQWNS SDYLHLNPMN FAIQTGKGGK YTVPGKVTLE DLQTFSDKFY CSCYANINCK KRVDIKNVHS VNVCMAEDIC IEGPVKLQPS DHSSSQNEAS TTTVSSISPS TTATTVSPCT PEKQSPECLK VRCLEAIANV TQTGCQGVKW KNTSSQSSIQ NIKNQTTY | 1 |
| Example 1-2 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY TVPGKVTLED LQTFSDKFYC SCYANINCKK RVDIKNVHSV NVCMAEDICI EGPVKLQPSD HSSSQNEAST TTVSSISPST TATTVSPCTP EKQSPECLKV RCL | 2 |
| Example 1-3 (BMI2004) | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY TVPGKVTLED LQTFSDKFYC SCYANINCKK RVDIKNVHSV NVCMAEDICI EGPVKLQPSD HSSSQNEAS | 3 |
| Example 1-4 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY TVPGKVTLED LQTFSDKFYC SCYANINCKK RVDIK | 4 |
| Example 1-5 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCHDEG VCTRKQWNSS DYLHLNPMNF AIQTGKGGKY T | 5 |
| Example 1-6 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTMQSCMNLG NYLNTTLNPY IINVTLAAKM CSQVLCH | 6 |
| Example 1-7 | DFRAPPLISN TSFLWAWNAP AERCVKIFKL PPDLRLFSVK GSPQKSATGQ FITLFYADRL GYYPHIDEKT GNTVYGGIPQ LGNLKNHLEK AKKDIAYYIP NDSVGLAVID WENWRPTWAR NWKPKDVYRD ESVELVLQKN PQLSFPEASK IAKVDFETAG KSFMQETLKL GKLLRPNHLW GYYLFPDCYN HNYNQPTYNG NCSDLEKRRN DDLDWLWKES TALFPSVYLN IKLKSTPKAA FYVRNRVQEA IRLSKIASVE SPLPVFVYHR PVFTDGSSTY LSQGDLVNSV GEIVALGASG IIMWGSLNLS LTM | 7 |

### EXAMPLE 2. Assay for Stability of Hyaluronidases According to Deletion Site

### (1) Comparison of enzymatic activity or content by storage pH according to deletion site

Among the hyaluronidases prepared in Example 1, six hyaluronidases, except for the hyaluronidase of Example 1-7 which lacks activity, were assayed for pH stability according to deletion site. Each amino acid sequence was expressed in transient cells and the cell cultures thus obtained were concentrated in the same manner before measuring enzymatic activity. The experiment concentration was adjusted into 1,500 IU/mL (=100%), solutions were prepared according to the conditions shown in Table 2 by substituting with water, and stored at 37°C for 4 weeks under the condition of pH 3.0, pH 5.0, pH 7.0, or pH 10.0 while titration was conducted every two weeks.

The specimens with different deletion sites were assayed for enzymatic activity or content at each time point according to the following protocols:
1) The activity of hyaluronidases with different deletion sites was determined by proportion comparison with EP STD.
1-1) Preparation of pH 6.4 phosphate buffered saline: a solution of 2.5 g of disodium hydrogen phosphate dodecahydrate, 2.5 g of sodium phosphate dihydrogen, and 8.2 g of sodium chloride in 950 mL of water was adjusted with 1 M sodium hydroxide or 1 M hydrochloride to have a pH of 6.4 and added with water to form a final volume of 1,000 mL.
1-2) Preparation of dilution: In a mixture of 100 mL of pH 6.4 phosphate-buffered saline and 100 mL of water, 0.140 g of a gelatin reagent was dissolved at 37°C. The dilution should be used within 2 hours after preparation.
1-3) Preparation of substrate solution: To 0.5 g of sodium hyaluronate, 100 mL of water was added little by little while stirring. Water was slowly added until sodium hyaluronate swelled. At 4°C, stirring was continued for 12 hours or longer. The substrate solution thus obtained was stored at 4°C and should be used within 4 days after preparation.
1-4) Preparation of standard solution: EP STD (EDQM) reference standard was dissolved in the dilution to give a concentration of about 50 IU/mL. Exact 3 mL of this solution was taken and put in a 250 mL volumetric flask to which the dilution was then added to form an exact volume of 250 mL. The resulting solution was used as a standard solution.
1-5) Preparation of test solution: The hyaluronidase test solutions with different deletion sites, each having about 0.6 IU/mL, were adjusted into the predetermined pH values and then diluted before use.

2) Manual: The standard and test solutions were tested according to the following method.
2-1) In a water bath maintained at 37°C, a 50-mL conical tube where 7.5 mL of pH 6.4 phosphate-buffered saline and 5.0 mL of the substrate solution had been mixed was left to stand.
2-2) To the conical tube containing pH 6.4 phosphate-buffered saline and the substrate solution, 2.5 mL of a test solution was added and mixed for 1 minute.
2-3) To a Ubbelohde microviscometer (DIN 51 562, Part 2, capillary type MIII, constant: about 0.1 mm²/s² or an equivalent viscometer), all of the mixed solution in the conical tube was loaded.
2-4) The time taken for the solution to flow down from the upper indication line to the lower indication line in the Ubbelohde microviscometer was recorded using a second chronometer.
2-5) The time was recorded several times during about 20 minutes.
2-6) The above procedure was repeated three times.

3) Calculation: Titers (IU/mg) were calculated according to the equation.
3-1) Reaction time: T₁ + T₂ / 2
3-2) ηr⁻¹ : {(k x T₂) / 0.6915}⁻¹
T₁ : time taken for the solution to ascend to the upper indication line in the Ubbelohde microviscometer (second)
T₂ : T - T₁
T : time taken for the solution to descend to the lower indication line in the Ubbelohde microviscometer (second)
k : Ubbelohde microviscometer constant (mm²/s²), refer to the specification of Ubbelohde microviscometer
0.6915: the kinematic viscosity of the substrate solution at 37°C (mm²/s²)
3-3) Activity calculation: (B_{T}/B_{R})*(E_{R}/E_{T})*A
B_{T} : slopes of the regression curves plotted for test solutions in a coordinate with reaction time on the x axis and natural log ηr⁻¹ on the y axis
B_{R} : slope of the regression curve plotted for standard solution in a coordinate with reaction time on the x axis and natural log ηr⁻¹ on the y axis
E_{T} : concentration of test solution (mg/mL)
E_{R} : concentration of standard solution (mg/mL)
A : titer of standard solution (IU/mg)

**TABLE 2**

| Specimen | Activity | Amino acid at cleaved site | Proportion of deleted enzyme region (%) | Storage pH | Storage Temp. |
|---|---|---|---|---|---|
| Example 1-1 | | L - Y | about 0% | | |
| Example 1-2 | | D - L | about 6% | | |
| Example 1-3 | (Transient Hyaluronidase Cell) | D - S | about 12% | | |
| Example 1-4 | | D - K | about 18% | pH 7.0 | 37 °C |
| Example 1-5 | | D - T | about 24% | | |
| Example 1-6 | | D - H | about 30% | | |
| Example 1-7 | | D - M | about 36% | | |

Test data for pH stability are shown in Table 3, below. The enzyme activity (in IU/mL) in each test is expressed as percentages of the reference value 1,500 IU/mL (=100%), which is the initial enzyme activity at week 0. The enzyme activity ratio was calculated according to the following equation: $Enzyme Activity Ratio \left(%\right) = \left(enzyme activity at measured time\right) / \left(initial enzyme activity\right) * 100$

**TABLE 3**

| Enzyme Activity Ratio (%) | pH 3.0 | | | pH 5.0 | | | pH 7.0 | | | pH 10.0 | | |
|---|---|---|---|---|---|---|---|---|---|---|---|---|
| Specimen | Week 0 | Week 2 | Week 4 | Week 0 | Week 2 | Week 4 | Week 0 | Week 2 | Week 4 | Week 0 | Week 2 | Week 4 |
| Example 1-1 | 100.47 | 61.31 | 31.62 | 100.43 | 72.84 | 55.30 | 100.36 | 73.17 | 56.03 | 100.88 | 63.31 | 45.92 |
| Example 1-2 | 102.00 | 84.77 | 55.05 | 101.17 | 94.52 | 80.57 | 101.47 | 95.88 | 81.24 | 101.25 | 86.29 | 64.98 |
| Example 1-3 | 102.66 | 85.48 | 55.26 | 101.80 | 95.02 | 81.19 | 101.49 | 96.22 | 82.55 | 102.68 | 89.95 | 65.11 |
| Example 1-4 | 101.23 | 84.91 | 54.76 | 101.19 | 94.96 | 80.65 | 101.38 | 95.50 | 81.73 | 101.49 | 88.27 | 64.19 |
| Example 1-5 | 100.46 | 83.59 | 52.94 | 100.15 | 94.88 | 77.45 | 100.60 | 93.87 | 78.81 | 100.67 | 86.74 | 62.64 |
| Example 1-6 | 100.82 | 82.32 | 50.58 | 100.08 | 92.61 | 74.88 | 99.32 | 91.36 | 77.63 | 99.75 | 85.76 | 61.17 |

As shown in Table 3, the pH stability data obtained for hyaluronidases with different deletion sites for 4 weeks indicates higher enzyme activity ratios (%) in the pH range of 5.0 to 7.0 than the other pH values. The hyaluronidases of Examples 1-2 to 1-6 exhibited remarkably high pH stability compared to the wild-type hyaluronidase of Example 1-1, with superiority of the hyaluronidases of Examples 1-2 to 1-4 to the other hyaluronidases. Values exceeding 100% seemed to be measurement errors that generally occurs due to large fluctuations in the titer tests of biological products such as vaccines and recombinant proteins (cytokines, monoclonal antibodies, etc.).

### (2) Comparison of enzyme activity or content by storage temperature according to deletion site

The hyaluronidases of Examples 1-1 to 1-6 were assayed for temperature stability in the same manner as in (1) of Example 2. Solutions were prepared according to the conditions listed in Table 2 and stored at a frozen temperature (-20°C), a refrigerated temperature (5°C), and a high temperature (40°C) for 4 weeks during which a titer test was conducted every two weeks. The hyaluronidases with different truncated sites were measured for stability against temperature for 4 weeks and the measurements are shown in Table 4.

**TABLE 4**

| Enzyme Activity Ratio (%) | Frozen | | | Refrigerated | | | High temperature | | |
|---|---|---|---|---|---|---|---|---|---|
| Specimen | Week 0 | Week 2 | Week 4 | Week 0 | Week 2 | Week 4 | Week 0 | Week 2 | Week 4 |
| Example 1-1 | 100.22 | 81.47 | 62.51 | 100.54 | 90.89 | 82.55 | 98.13 | 65.09 | 51.55 |
| Example 1-2 | 101.98 | 100.74 | 98.85 | 101.52 | 100.81 | 99.45 | 98.96 | 88.92 | 78.54 |
| Example 1-3 | 102.68 | 100.88 | 99.22 | 102.77 | 101.91 | 100.23 | 99.47 | 89.90 | 79.63 |
| Example 1-4 | 101.07 | 100.17 | 98.03 | 100.32 | 100.72 | 99.18 | 98.16 | 88.11 | 77.95 |
| Example 1-5 | 100.12 | 98.82 | 95.52 | 100.53 | 98.70 | 97.51 | 99.39 | 85.31 | 75.78 |
| Example 1-6 | 99.49 | 96.92 | 93.12 | 99.64 | 96.32 | 95.63 | 99.87 | 82.69 | 73.12 |

As shown in Table 4, the hyaluronidases of Examples 1-2 to 1-6 exhibited great temperature stability and retained remarkably high enzyme activity ratios (%) particularly in the frozen and refrigerated storage conditions. The amino acid D-S hyaluronidase corresponding to Example 1-3 which was truncated by about 12%, showing the highest activity among the recombinant hyaluronidases, was named BMI2004 and used in the experiments.

### EXAMPLE 3. Assay for Stability of Wild-Type Hyaluronidase and BMI2004

### (1) Comparison of enzyme activity or content according to pH

Comparison was made of pH stability between BMI2004 prepared in Example 1 and a wild-type hyaluronidase. The BMI2004 and wild-type hyaluronidase (manufacturer; Korea BMI, tradename; Hirax, hereinafter referred to as Hirax) used in the pH stability test were 95% or higher in purity and each had a concentration of 1,500 IU/mL (=100%), solutions were prepared according to the conditions in Table 5 by substituting with water, and stored at 5°C under the conditions of pH 3.0, pH 5.0, pH 7.0, and pH 10.0 for 4 weeks during which a titer test was conducted every two weeks. At each time point, the specimens were measured for enzyme activity or content in the same manner as in (1) of Example 2.

**TABLE 5**

| pH | Specimen | Storage Temp. |
|---|---|---|
| 3.0 | | 2-8°C |
| 5.0 | BMI2004 / Hirax (1,500 IU/mL) | 2-8°C |
| 7.0 | | 2-8°C |
| 10.0 | | 2-8°C |

Measurements of the pH stability assay are shown in Table 6. The enzyme activity (in IU/mL) in each test is expressed as percentages of the reference value 1,500 IU/mL (=100%), which is the initial enzyme activity at week 0. The enzyme activity ratio was calculated according to the following equation: $Enzyme Activity Ratio \left(%\right) = \left(enzyme activity at measured time\right) / \left(initial enzyme activity\right) * 100$

**TABLE 6**

| Enzyme Activity Ratio (%) | pH 3.0 | | | pH 5.0 | | | pH 7.0 | | | pH 10.0 | | |
|---|---|---|---|---|---|---|---|---|---|---|---|---|
| Specimen | Week 0 | Week 2 | Week 4 | Week 0 | Week 2 | Week 4 | Week 0 | Week 2 | Week 4 | Week 0 | Week 2 | Week 4 |
| BMI2004 | 102.66 | 85.48 | 55.26 | 101.80 | 95.02 | 81.19 | 101.49 | 96.22 | 82.55 | 102.68 | 89.95 | 65.11 |
| Hirax | 102.04 | 51.11 | 31.03 | 103.39 | 71.20 | 50.40 | 102.33 | 70.76 | 50.95 | 101.88 | 62.07 | 41.03 |

As shown in Table 6, stability of BMI2004 and Hirax according to pH were observed during about 4 weeks. Higher enzyme activity ratios (%) were measured at pH 5.0 and pH 7.0 than pH 3.0 and pH 10.0. After week 2, the content of Hirax (%) tended to rapidly decrease, compared to BMI2004. Values exceeding 100% seemed to be measurement errors that generally occurs due to large fluctuations in the titer tests of biological products such as vaccines and recombinant proteins (cytokines, monoclonal antibodies, etc.).

In order to additionally test the change in enzyme activity (or content) for stability confirmation according to pH of Hirax and BMI2004, SDS-PAGE (Sodium Dodecyl Sulfate-PolyAcrylamide Gel Electrophoresis) was performed under the conditions of Table 5 above, and according to the following protocol:
1) Preparation of sample buffer (5X): Pierce^{™} Lane Marker Reducing Sample Buffer (Thermo Scientific^{™}, Cat No. 39000) was used.
2) Preparation of running buffer (1X): Novex^{™} Tris-Glycine SDS Running Buffer (10X) (Invitrogen, Cat No. LC2675) was used. Exact 100 mL of the solution was taken and put in a 1,000-mL volumetric flask to which water was then added to form an exact volume of 1,000 mL.
3) Preparation of test solution: reference standards for Hirax and BMI2004 were modified with water according to the conditions. About 20 µL of each of the solutions and 5 µL of the sample buffer (5X) were exactly taken and mixed in an EP tube.
4) Manual: Test solutions and PageRuler Prestained Protein Ladder reference standard (Thermo, Cat No. 26616) were subjected to the following assays.
   4-1) After removal of a comb therefrom, Novex^{TM} Wedgewell^{TM} 8~16% Tris-Glycine Gel (Invitrogen, Cat No. XP08160BOX) or a sheet of equivalent gel in was cleansed with water to substitute for the buffer.
   4-2) The washed gel was installed into the Mini Gel Tank. In the Mini Gel Tank, the cathode compartment was fully filled with running buffer (1X) while the anode compartment was filled about 2/3 with the running buffer (1X).
   4-3) Into the gel, 7 µL of PageRuler Prestained Protein Ladder standard and 25 µL of the test solution were loaded.
   4-4) The Mini Gel Tank was connected to a power supply and manipulated as follows, after which the specimens were run to 90% of the gel length:
      Volt.: 140 V
      Ampere: 400 mA
      Time: 60 minutes (the time may be changed depending on the running condition.)
   4-5) After completion of the running, the gel was separated from the cast and washed with water.
   4-6) The washed gel was immersed in a tray containing a staining reagent (Coomassie Brilliant Blue R-250 Staining Solution, BIO-RAD, Cat No. 1610436).
   4-7) The tray was put on a Rocker and shaken at 30 rpm for 30 minutes.
   4-8) After completion of the staining, the gel was transferred to a tray containing a destaining solution (Coomassie Brilliant Blue R-250 Destaining Solution, BIO-RAD, Cat No. 1610438) and the tray was put on a rocker and shaken at 30 rpm until the gel was destained while the destaining solution was changed with a fresh one. When the destaining was conducted to some extent, the gel was immersed in water to remove the destaining solution.
   4-9) When the destaining solution was removed therefrom, the gel was observed on a white lamp.

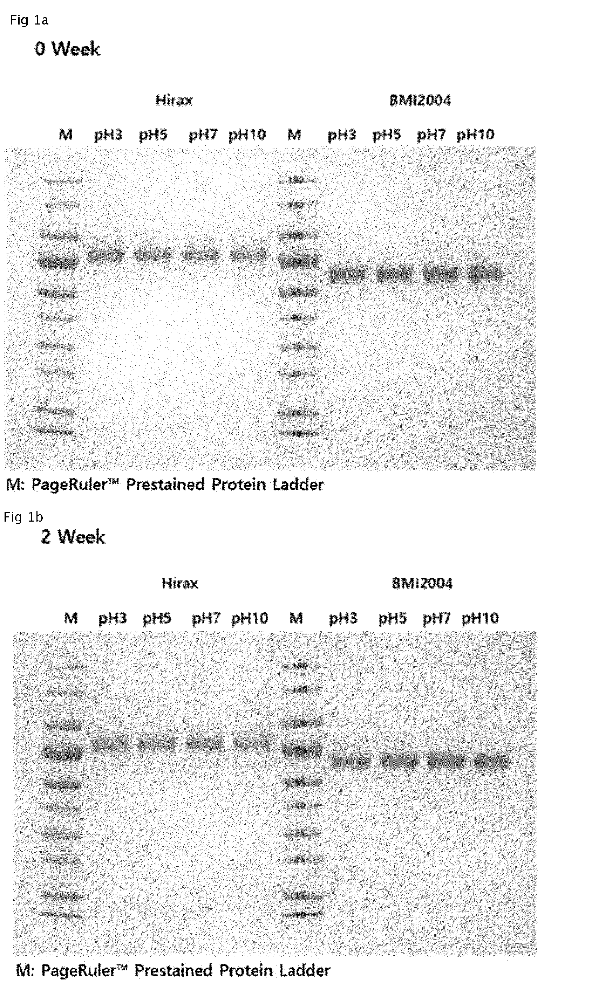

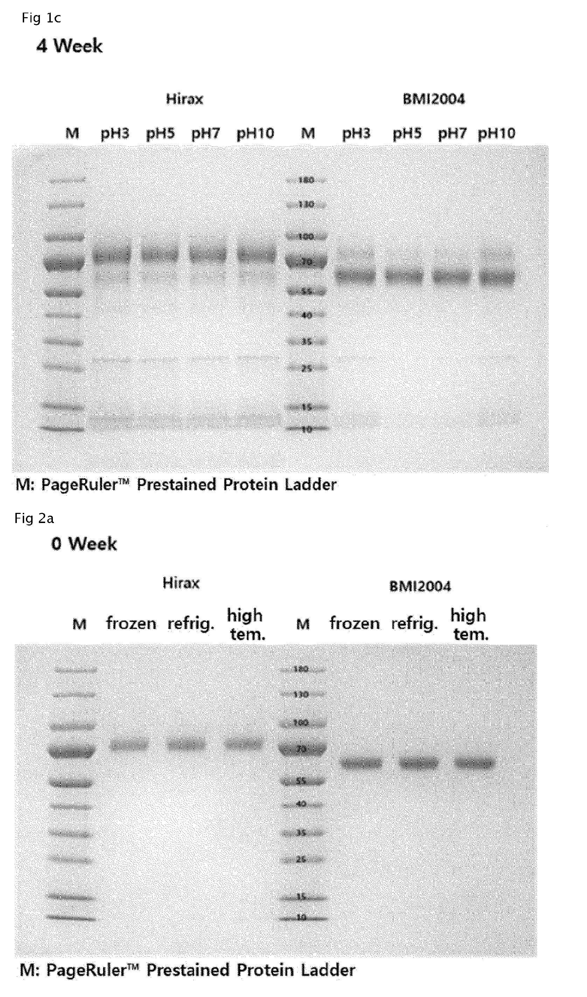

The test results are depicted in FIG. 1a to FIG. 1c. As shown in FIG. 1a to FIG. 1c, it could be understood from whether or not other band is generated according to time flow in the state of being replaced with water on SDS-PAGE results that BMI2004 was more stable against pH with time than Hirax.

### (2) Comparison of enzyme activity or content according to storage temperature

Comparison was made of temperature stability between BMI2004 prepared in Example 1 and Hirax. The BMI2004 and Hirax used in the temperature stability test were 95% or higher in purity and each had a concentration of 1,500 IU/mL (=100%). Solutions were prepared using water according to the conditions in Table 7 and stored at a frozen temperature (-20°C), a refrigerated temperature (5°C), and a high temperature (40°C) for 4 weeks during which a titer test was conducted every two weeks.

**TABLE 7**

| Storage Temp. | Specimen | Storage pH |
|---|---|---|
| ≤-20 °C | Hirax / BMI2004 (1,500 IU/mL) | pH 5.0 |
| 2-8 °C | | pH 5.0 |
| ≥40 °C | | pH 5.0 |

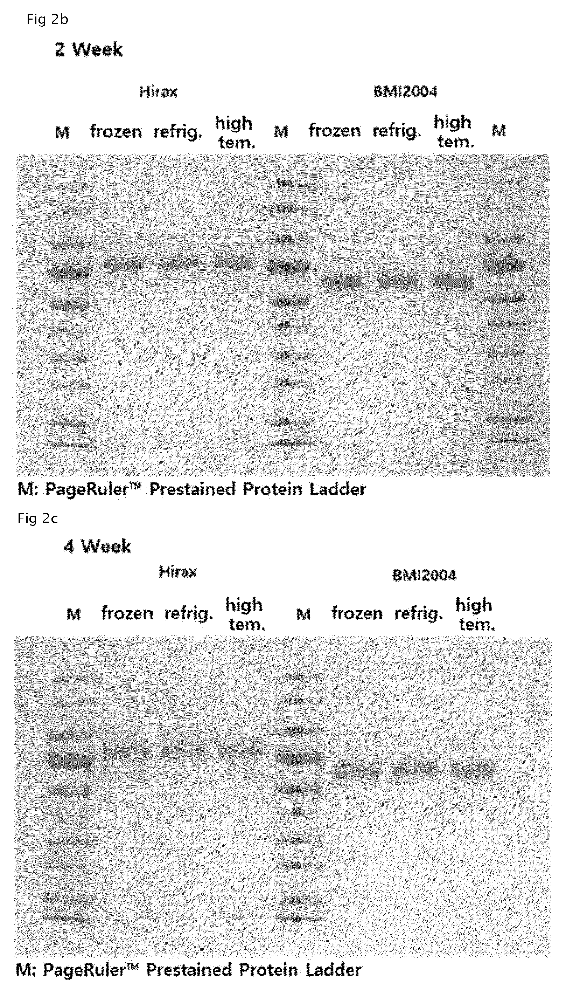

Hirax and BMI2004 were measured for stability against temperature for 4 weeks and the measurements are summarized in Table 8 and depicted in FIG. 2. The highest contents (%) were detected at frozen temperatures of -20°C or less. After week 2, the content (%) of BMI2004 was maintained significantly high relative to Hirax in all temperature conditions.

**TABLE 8**

| Enzyme activity ratio (%) | Frozen | | | Refrigerated | | | High temperature | | |
|---|---|---|---|---|---|---|---|---|---|
| Specimen | Week 0 | Week 2 | Week 4 | Week 0 | Week 2 | Week 4 | Week 0 | Week 2 | Week 4 |
| BMI2004 | 102.68 | 100.88 | 99.22 | 102.77 | 101.91 | 100.23 | 99.47 | 89.90 | 79.63 |
| Hirax | 102.76 | 80.24 | 59.75 | 101.42 | 91.51 | 82.83 | 102.19 | 66.40 | 40.15 |

In an additional stability assay according to temperature, Hirax and BMI2004 were measured for content changes at various temperature in the same manner as in Example 3. In this regard, SDS-PAGE was conducted in the conditions of Table 7.

Results of SDS-PAGE are depicted in FIG. 2a to FIG. 2c. As shown in FIG. 2a to FIG. 2c, it was understood from the formation of other bands positioned other than the initial position of the main band at frozen, refrigerated, and high temperatures with time in an aqueous condition, that BMI2004 was more stable than Hirax.

### EXAMPLE 4. Optimal Enzyme Activity According to Temperature

A plate titer assay in this Example was designed on the basis of the cylinder plate assay among microbial assays for antibiotics. Specifically, peni-cylinders having a predetermined amount of hyaluronidase distributed therein were placed on solid agarose plates containing hyaluronic acid to allow the hyaluronidase to diffuse across the agarose plates. Through this diffusion, the hyaluronic acid was degraded by hyaluronidase, and the hyaluronic acid remaining undegraded were precipitated by cetylpyridinium chloride to form clear halos. In this Example, the enzymatic activity of hyaluronidase could be thus determined by the size of the halo generated through such a simple method using plates. In each condition, the plate titer assay was conducted as follows:
1) Preparation of hyaluronic acid solution: 0.2 g of hyaluronic acid was completely dissolved in 100 mL of water and the pH was adjusted with hydrochloric acid and sodium hydroxide to a pH of 7.0±0.1.
2) Preparation of 1.5% agarose: 1.5 g of agarose (SIGMA, Cat No. A9539) was added to 100 mL of water and dissolved with microwaves.
3) Preparation of 10% cetylpyridinium chloride: 10 g of cetylpyridinium chloride (SIGMA, Cat No. C0732) was dissolved in 100 mL of water.
4) Test solution: Hirax and BMI2004 reference standards were adjusted with water according to the conditions.
5) Manual: Test solutions were subjected to the following assays.
   5-1) The hyaluronic acid solution was warmed at 37°C for about 20 minutes before mixing with 1.5% agarose.
   5-2) After 100 mL of 1.5% agarose was cooled to about 60°C, 100 mL of the hyaluronic acid solution was added and mixed by stirring.
   5-3) The mixture was poured to a thickness of about 3 mm in a petri dish (SPL, Cat No. 10050).
   5-4) After the agarose gel was fully solidified, the peni cylinder (KisanBio, Cat No. KS-P0161) was placed at an angle of 90° on the concentric circle of the petri dish. The test solution was taken in an amount of 20 µL and loaded into the peni cylinder, followed by incubation for 18-20 hours in an incubator maintained at the temperature conditions listed in each table.
   5-5) After completion of the reaction, the peni cylinder was removed and 3 mL of 10% cetylpyridinium chloride was added. After 20 minutes, appearance of clear halos was monitored.
   5-6) The halo was measured precisely to an extent of 0.5 mm or less for diameter (mm).

### (1) Confirmation of Activity of hyaluronic acid solution

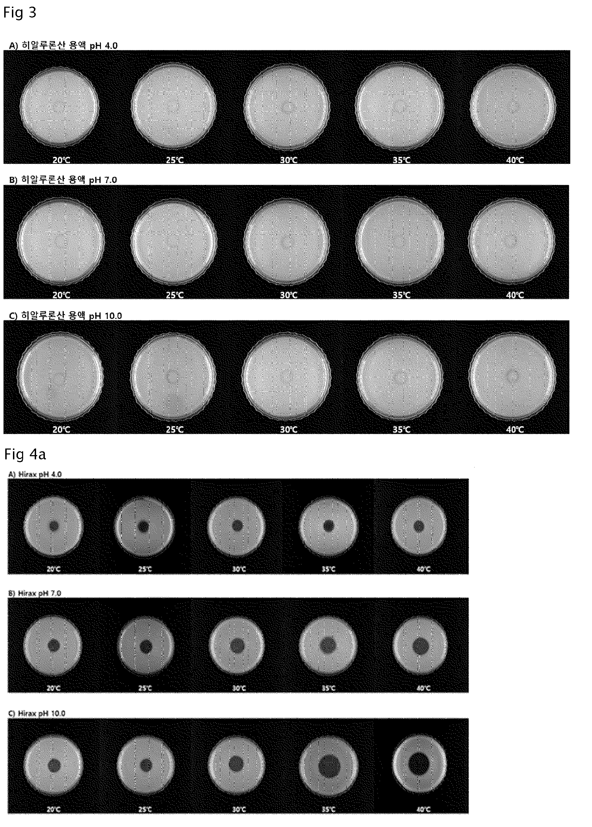

In order to examine whether hyaluronic acid solutions exhibited their own activity, plate titer tests were carried out at various pH values and temperature conditions before an assay for optimal enzymatic activity. The tests were carried out at 20°C, 25°C, 30°C, 35°C, and 40°C under the condition of pH 4.0, pH 7.0, or pH 10.0. The test results are depicted in FIG. 3. As shown in FIG. 3, the hyaluronic acid solutions were observed to have no their own activity.

### (2) Comparison of enzymatic activity in temperature condition of 20-40°C

The BMI2004 and Hirax used in this assay for enzymatic activity were 95% or higher in purity and were each adjusted with water to have a concentration of 1,500 IU/mL (=100%). Solutions were prepared according to the conditions in Table 9 and subjected to plate titer assays at 20°C, 25°C, 30°C, 35°C, and 40°C.

**TABLE 9**

| | Storage pH | Storage Temp. | Specimen |
|---|---|---|---|
| Condition 1 | pH 4.0 | 20-40 °C | Hirax / BMI2004 (1,500 IU/mL) |
| Condition 2 | pH 7.0 | 20-40 °C | |
| Condition 3 | pH 10.0 | 20-40 °C | |

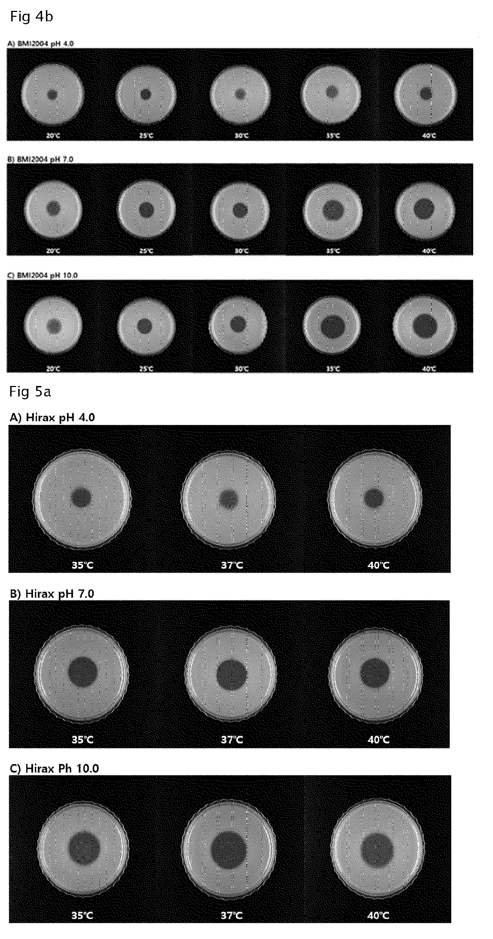

Assay results are depicted in FIG. 4a and FIG. 4b. Enzymatic activity of Hirax and BMI2004 was examined by a plate titer assay at 20 to 40°C. As a result, the halos appeared large at 35°C and 40°C.

To quantitate enzymatic activity, diameters of the halos in FIG. 4a and FIG. 4b were measured and the measurements are shown in Table 10, below. In Table 10, the diameters (mm) had an error deviation of ±0.5 mm.

**TABLE 10**

| Temp. | Specimen | pH 4.0 | pH 7.0 | pH 10.0 |
|---|---|---|---|---|
| 20°C | Hirax | 8 mm | 10 mm | 11 mm |
| | BMI2004 | 8 mm | 12 mm | 13 mm |
| 25°C | Hirax | 9 mm | 10 mm | 11 mm |
| | BMI2004 | 9 mm | 13 mm | 13 mm |
| 30°C | Hirax | 9 mm | 13 mm | 14 mm |
| | BMI2004 | 10 mm | 13 mm | 15 mm |
| 35°C | Hirax | 10 mm | 15 mm | 18 mm |
| | BMI2004 | 11 mm | 18 mm | 21 mm |
| 40°C | Hirax | 10 mm | 15 mm | 18 mm |
| | BMI2004 | 11 mm | 18 mm | 21 mm |

As shown in Table 10, comparison of enzymatic activity at 20 to 40°C indicated that the activity of BMI2004 was higher than that of Hirax.

### (3) Comparison of enzymatic activity in temperature condition of 35-40°C

On the basis of the results of activity comparison between Hirax and BMI2004 measured in (2) of Example 4, additional tests were conducted in subdivided temperature conditions. In this regard, solutions were prepared according to the conditions listed in Table 11 below and subjected to plate titer assays at 35°C, 37°C, and 40°C.

**TABLE 11**

| | Storage pH | Storage Temp. | Specimen |
|---|---|---|---|
| Condition 1 | pH 4.0 | 35°C | |
| | | 37°C | |
| | | 40°C | |
| Condition 2 | pH 7.0 | 35°C | Hirax / BMI2004 (1,500 IU/mL) |
| | | 37°C | |
| | | 40°C | |
| Condition 3 | pH 10.0 | 35°C | |
| | | 37°C | |
| | | 40°C | |

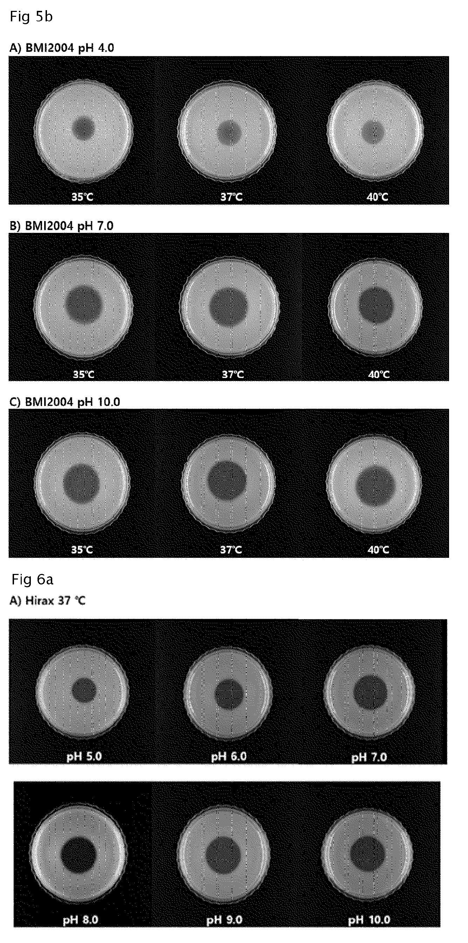

The test results are shown in FIG. 5a and FIG. 5b. Enzymatic activity of Hirax and BMI2004 was examined by a plate titer assay at 35°C, 37°C, and 40°C. As a result, the halos appeared largest at 37°C. Diameters of the halos were shown in Table 12, below. The diameters (mm) had an error deviation of ±0.5 mm.

**TABLE 12**

| Specimen | 35°C | | 37°C | | 40°C | |
|---|---|---|---|---|---|---|
| | Hirax | BMI2004 | Hirax | BMI2004 | Hirax | BMI2004 |
| pH 4.0 | 10 mm | 12 mm | 10 mm | 13 mm | 10 mm | 12 mm |
| pH 7.0 | 16 mm | 19 mm | 17 mm | 20 mm | 16 mm | 19 mm |
| pH 10.0 | 18 mm | 21 mm | 19 mm | 22 mm | 18 mm | 21 mm |

As shown in Table 12, comparison of enzymatic activity at 35°C, 37°C, and 40°C indicated that the activity of BMI2004 was higher than that of Hirax.

### EXAMPLE 5. Optimal Enzymatic Activity According to pH

The Hirax and BMI2004 used in the assay for optimal enzymatic activity were 95% or higher in purity and were each adjusted with water to have a concentration of 1,500 IU/mL (=100%). Solutions were prepared according to the conditions in Table 13. The enzymatic activity was measured according to the assay of Example 4. On the basis of the activity result according to temperature measured in (3) of Example 4, a plate titer assay according to pH was conducted in the condition of 37°C at which the largest halo appeared.

**TABLE 13**

| | Storage pH | Storage Temp | Specimen |
|---|---|---|---|
| Condition 3 | pH 5.0 | | |
| Condition 4 | pH 6.0 | | |
| Condition 5 | pH 7.0 | 37 °C | Hirax / BMI2004 (1,500 IU/mL) |
| Condition 6 | pH 8.0 | | |
| Condition 7 | pH 9.0 | | |
| Condition 8 | pH 10.0 | | |

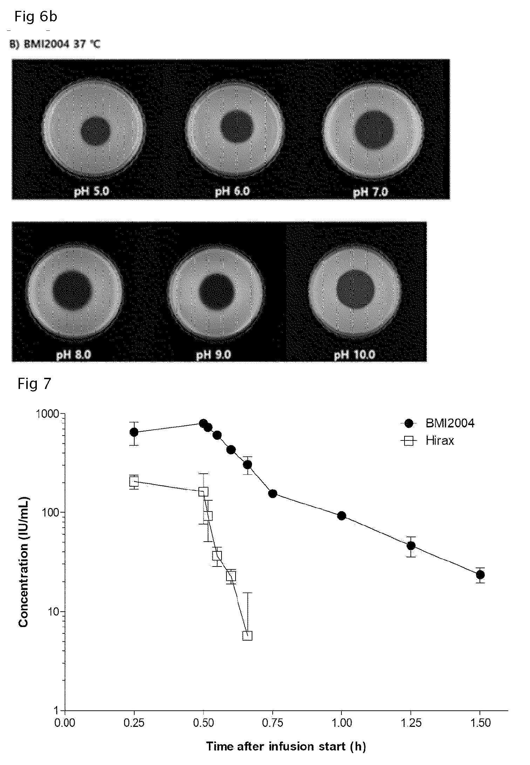

Assay results are depicted in FIG. 6a and FIG. 6b. As a result of the plate titer assay for enzymatic activity of Hirax and BMI2004 at 37°C under the condition of pH 5.0-10.0, the halos appeared particularly large at a pH of 7.0-10.0. Comparison of halo diameters are shown in Table 14 below. The diameter (mm) had an error deviation of ±0.5 mm.

**TABLE 14**

| Specimen | 37 °C | |
|---|---|---|
| | Hirax | BMI2004 |
| pH 5.0 | 14 mm | 16 mm |
| pH 6.0 | 17 mm | 18 mm |
| pH 7.0 | 19 mm | 20 mm |
| pH 8.0 | 19 mm | 20 mm |
| pH 9.0 | 19 mm | 20 mm |
| pH 10.0 | 20 mm | 21 mm |

As can be seen in Table 14, comparison of enzymatic activity at 37°C under the condition of pH 5.0-10.0 indicated that the activity of BMI2004 was superior to that of Hirax at a pH of 5.0 or higher, and particularly superior at a pH of 7.0 or higher.

### EXAMPLE 6. Assay for Titer of Hyaluronidase

The hyaluronidase according to an embodiment of the present disclosure and the conventional enzyme Hirax were measured for titer in the same manner as in (1) of Example 2, and the measurements are shown in Table 15.

**TABLE 15**

| Sample no. | BMI2004 (IU/mg) | Hirax (IU/mg) |
|---|---|---|
| 1 | 124,497 | 81,396 |
| 2 | 133,870 | 81,528 |
| 3 | 134,678 | 80,653 |
| 4 | 122,433 | 80,538 |
| Average | 128,870 | 81,029 |

As shown in Table 15, BMI2004 exhibited an activity of about 122,433 to 134,678 IU per mg of protein while Hirax exhibited an activity of about 80,538 to 81,528 IU per mg of protein. Therefore, the hyaluronidase according to an embodiment of the present disclosure could achieve equivalent activity even when used in an amount of about 63 % of the amount of conventional Hirax.

### EXAMPLE 7. Promotive Effect of Hyaluronidase on Drug Absorption and Diffusion (1)

In order to confirm the promotive effect on drug absorption and diffusion of the hyaluronidase according to an embodiment of the present disclosure, an assay for drug absorption and diffusion promotion was carried out using Trypan Blue.

Specifically, Hirax and BMI2004 were each mixed with 0.2% Trypan blue solution and injected subcutaneously once into Balb/c nude mice which were monitored for the diffusion of trypan blue. On the basis of the titers listed in Table 15, all the drugs were formulated to have a concentration or 10 IU/mL or 100 IU/mL under the conditions of Table 16 and administered at a dose of 0.05 mL. At each time, the diffused areas (πmm²) were measured.

**TABLE 16**

| Specimen (IU/mL) | Peptide dose (ug/mL) | 2.5 min | 5 min | 10 min | 15 min | 20 min |
|---|---|---|---|---|---|---|
| Saline | 0 | 77.38± 15.86 | 84.61± 13.92 | 98.11± 7.83 | 117.67± 10.35 | 121.84± 11.48 |
| Hirax (10 IU/mL) | about 0.123 | 74.45± 22.40 | 112.84± 15.01* | 146.69± 29.36** | 165.98± 18.49* | 189.90± 41.85** |
| Hirax (100 IU/mL) | about 1.234 | 84.25± 15.81 | 133.90± 14.52*** | 149.27± 27.41** | 184.80± 37.10** | 215.12± 20.60*** |
| BMI2004 (10 IU/mL) | about 0.078 | 82.70± 22.95 | 109.65± 12.70* | 153.17± 15.30*** | 176.85± 14.08** | 187.63± 36.40** |
| BMI2004 (100 IU/mL) | about 0.776 | 87.02± 17.51 | 126.70± 16.00*** | 140.23± 16.95*** | 180.98± 29.93*** | 218.14± 26.85*** |

| | | | | | | |
|---|---|---|---|---|---|---|
| 1) Mean±SD 2) *p < 0.05, **p < 0.01, ***p < 0.001 compared to saline treated group by SPSS (one-way ANOVA, LSD test) | | | | | | |

As shown in Table 16, the diffused areas for 2.5 to 20 minutes were measured. From 5 minutes after administration, Hirax and BMI2004 significantly increased the diffused areas compared to the negative control saline.

### EXAMPLE 8. Promotive Effect of Hyaluronidase on Drug Absorption and Diffusion (2)

In order to confirm the promotive effect on drug absorption and diffusion of the hyaluronidase according to an embodiment of the present disclosure, an assay for drug absorption and diffusion promotion was carried out using an akinesia model.

Specifically, Cynomolgus Monkeys were used as Akinesia Models for examining ability to increase the penetration of drugs. Test materials were BMI2004 and Hirax listed in Table 15 while saline was used as a negative control. Lidocaine and bupivacaine were used as anesthetics to be mixed with the specimens. The specimens were each injected at a dose of 2 mL to 6 monkeys through the right and the left peribulbar route.

After administration of the anesthetics along with the specimens, the time until the movement of the pupil disappeared (time to akinesia) and the time from the anesthesia of the pupil to the observation of pupil movement (duration of akinesia) were measured and the measurements are shown in Table 17.

**TABLE 17**

| Specimen | Peptide dose (ug/mL) | Model (Monkey) | Eye | Time to Akinesia (min) | Duration of Akinesia (min) |
|---|---|---|---|---|---|
| Saline | 0 | 02 | Left | No Akinesia | No Akinesia |
| | | 03 | Right | No Akinesia | No Akinesia |
| | | 05 | Left | No Akinesia | No Akinesia |
| | | 06 | Right | No Akinesia | No Akinesia |
| Hirax (500 IU/ 0.2 mL) | ca. 6.17*µ*g/ 0.2mL | 01 | Left | 4 | 27 |
| | | 02 | Right | 4 | 27 |
| | | 04 | Left | 2 | 17 |
| | | 05 | Right | 2 | 32 |
| | | Mean | | 3.0 | 25.8 |
| | | SD | | 1.2 | 6.3 |
| BMI2004 (500 IU/ 0.2 mL | ca. 3.88*µ*g/ 0.2mL | 01 | Right | 1 | 32 |
| | | 03 | Left | 2 | 24 |
| | | 04 | Right | 2 | 17 |
| | | 06 | Left | 1 | 41 |
| | | Mean | | 1.5 | 28.5 |
| | | SD | | 0.6 | 10.3 |

| | | | | | |
|---|---|---|---|---|---|
| 1) Drug; Lidocaine 2% (0.9 mL), Bupivacaine 0.5% (0.9 mL) and the dosing article (0.2 mL) | | | | | |

As seen in Table 17, the negative control saline exhibited no akinesia effects, while when administered together with the anesthetics, the polypeptide according to an embodiment of the present disclosure enhanced the penetration of the anesthetics and thus brought about an akinesia effect equivalent to that of the conventional peptide Hirax, demonstrating its ability to induce drug absorption and diffusion. In addition, the polypeptide according to an embodiment of the present disclosure exhibited equivalent activity at a protein dose of about 63% of that of conventional Hirax.

### EXAMPLE 9. Assay for Ability of Hyaluronidase to Promote Reabsorption of Excessive Body Fluid

In order to confirm the promotive effect on reabsorption of excessive body fluid, an edema model was employed. In this Example, the edema model was constructed by artificially causing lymphedema therein. Test materials were BMI2004 and Hirax listed in Table 15 while saline was used as a negative control. Specimens and doses are given in Table 18 below.

**TABLE 18**

| Specimen | Lymphedema | Dose (/site) | Route | No. of animal |
|---|---|---|---|---|
| Saline | + | - | SC | 6 |
| Hirax | + | 100 IU (ca. 1.234ug) | SC | 6 |
| BMI2004 | + | 100 IU (ca. 0.776ug) | SC | 6 |

Lymphedema induction was conducted in the tail due to accuracy and ease. An annular cut of the skin 2 mm wide was made 1 cm from the base of the tail of the mouse. In this regard, about 4 mm² of the ventral side was left without being cut out. In order to examine the effect of reducing edema, as listed in Table 19 below, the diameter (mm) of the tail was measured at each time point from pre-administration (0 hour) to day 15 of induction after the first administration. The dose was made secondarily 24 hours later and thirdly 48 hours later. In this test, the diameter (mm) of the tail was measured using a caliper at the 10 mm point of the defect site.

**TABLE 19**

| Specimen | Saline | Hirax | BMI2004 |
|---|---|---|---|
| 0 hr | 4.55±0.43 | 4.53±0.49 | 4.53±0.38 |
| 1 hr | 4.57±0.43 | 4.39±0.49 | 4.57±0.65 |
| 2 hrs | 4.48±0.36 | 4.45±0.40 | 4.50±0.37 |
| 4 hrs | 4.52±0.38 | 4.41±0.38 | 4.48±0.51 |
| 24 hrs | 4.56±0.34 | 4.57±0.39 | 4.55±0.45 |
| 25 hrs | 4.68±0.32 | 4.48±0.35 | 4.53±0.44 |
| 26 hrs | 4.56±0.32 | 4.43±0.38 | 4.45±0.47 |
| 28 hrs | 4.58±0.37 | 4.42±0.44 | 4.46±0.48 |
| 48 hrs | 4.65±0.39 | 4.49±0.41 | 4.47±0.38 |
| 49 hrs | 4.56±0.27 | 4.22±0.37 | 4.26±0.37 |
| 50 hrs | 4.58±0.25 | 4.13±0.38 | 4.09±0.36 |
| 52 hrs | 4.59±0.25 | 4.16±0.47 | 4.18±0.36 |

As shown in Table 19, the administration of BMI2004 reduced lymphedema, demonstrating the promotive effect of the protein on reabsorption of excessive body fluids. In addition, the polypeptide according to an embodiment of the present invention exhibited equivalent activity at a protein dose of about 63% compared to conventional Hirax.

### EXAMPLE 10. Assay for In-vivo Stability of Hyaluronidase

In order to confirm in-vivo stability, the hyaluronidase according to an embodiment of the present disclosure was intravenously injected and monitored for pharmacokinetics. In this Example, SD-rats were used as test animals. To the animals, Hirax and BMI2004 were each administered at a total dose of about 180,000 IU for 30 minutes by intravenous infusion. Blood samples were taken before administration, 15 minutes after initiation of the infusion (mid-infusion), 30 minutes after initiation of the infusion (end of infusion), and 31, 33, 36, 40, 45, 60, 75, 90 minutes, and 2.5 hours, 4.5 hours, 24.5 hours, and 48.5 hours after initiation of the infusion.

Pharmacokinetic variables of the polypeptide according to an embodiment of the present disclosure and Hirax are given in Table 20. The blood levels of the polypeptide over time are depicted in FIG. 7. As seen in FIG. 7 and Table 20, Hirax was degraded too rapidly to measure in-vivo half-life. In contrast, the polypeptide according to an embodiment of the present disclosure was observed to have a half-life of about 0.272 hours (about 16.3 minutes). With a longer half-life than Hirax, the polypeptide according to an embodiment of the present disclosure has the potential to show a greater effect than Hirax by staying in the body for a long time. In addition, they were different in terms of in-vivo blood level. The blood level of Hirax peaked within 0.25 hours (15 minutes), but the blood level of BMI2004 peaked at 0.5 hours (30 minutes) after administration. The polypeptide exhibited a potential to bring about a greater effect than Hirax as it more slowly increased in blood level and remained in vivo for a longer period of time than Hirax.

**TABLE 20**

| Test material | Dose (IU/kg) | tₘₐₓ (h) | t_{1/2} (h) | CL (mL/h/kg) |
|---|---|---|---|---|
| Hirax | 180000 | 0.250 | NC | NC |
| BMI2004 | 180000 | 0.500 | 0.272 | 418 |

### EXAMPLE 11. Preparation of drug for subcutaneous injection

A composition for subcutaneous injection comprising a hyaluronidase according to an embodiment of the present disclosure and a drug was prepared. BMI2004 of Example 1-3 was used as hyaluronidase and commercially available Herceptin was used as a drug. Specifically, the BMI2004 hyaluronidase of Example 1-3 was added to the formulation buffer (5mM sodium acetate hydrate, 1mM magnesium chloride hydrate, 0.9% sodium chloride, 1.33% lactose hydrate, 0.01% Polysorbate 80, pH 5.0) to a concentration of 2,000 IU/mL, and mixed with 120 mg of Herceptin Inj. (Trastuzumab, Roche Korea Co., Ltd.) powder to prepare a drug for subcutaneous injection at a ratio of Herceptin 120 mg/BMI2004 2,000 IU/mL.

### EXAMPLE 12. Effect of improving bioavailability of drug for subcutaneous injection

In order to verify the effect of promoting drug absorption in the body of the subcutaneous injection formulation prepared in Example 11, the pharmacokinetics were verified after subcutaneous injection. In this Example, mice (ICR, Female) were used as test animals, and in the single-injection study, Herceptin was injected to a total of 90 mice and blood was collected from 2 mice per time point.

As shown in Table 21, the administered dose was 6mg for Herceptin IV in Group 1 which is the control group 1, 6mg Herceptin/100IU rHuPH20 for Herceptin Hylecta in Group 2 which is the control group 2, and finally, 6mg Herceptin/100IU BMI2004 for Group 3 which is the test substance, and each was formulated and administered subcutaneously. Specifically, in the negative control group, which was administered drug alone (Group 1), 150 mg/vial powder of Herceptin Inj. (Trastuzumab, Roche Korea Co., Ltd.) was dissolved to 1.25 mL of the formulation buffer (5 mM sodium acetate hydrate, 1 mM magnesium chloride hydrate, 0.9% sodium chloride, 1.33% lactose hydrate, 0.01% Polysorbate 80, pH 5.0) to prepare a concentration of 120 mg/mL and administered at 0.05 mL per mouse. In the positive control group, Herceptin Hylecta administration group (Group 2), Herceptin Hylecta^{TM} 600 mg/10,000 IU/5 mL (120 mg/2,000 IU/mL, liquid) which is a commercially available Herceptin drug formulated with rHuPH20 hyaluronidase, was administered at 0.05 per mouse. Group 3, the experimental group, was administered 0.05 mL of Herceptin 120 mg/BMI2004 2,000 IU/mL subcutaneous injection drug prepared in Example 11 per mouse. blood was collected at each time point from 0 hours (before administration) to 0.17, 0.5, 0.83, 2, 4, 8, 12, 24, 48, 72, 96, 120, 144, 168, 192, 240, 336, 504, 672, and 840 hours (total of 21 time points), Herceptin concentration was measured using ELISA (Enzyme linked immunosorbent assay).

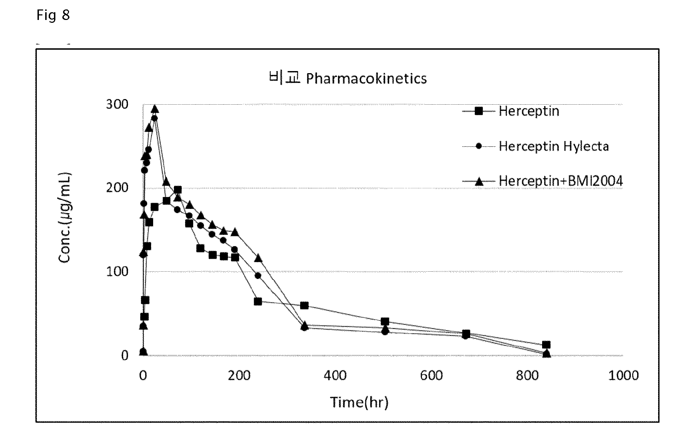

As shown in Table 22 and Figure 8, Herceptin to which hyaluronidase was added was absorbed into the body faster than Herceptin to which hyaluronidase was not added. In addition, the Herceptin drug formulated with hyaluronidase according to an embodiment of the present disclosure showed similar pharmacokinetic results to the commercially available Herceptin hylecta to which rHuPH20 hyaluronidase was added. Therefore, hyaluronidase according to an embodiment of the present disclosure can be used to formulate drugs for subcutaneous injection, and has the effect of promoting absorption of drugs into the body.

**TABLE 21**

| Group | Sample | Animal | Administration method | Dosage (Herceptin/Hyaluro nidase) | Blood collection time (hours) |
|---|---|---|---|---|---|
| 1 | Herceptin | ICR, Female | Subcutaneous injection | 6mg/0IU | 0 (Before administration), 0.17, 0.5, 0.83, 2, 4, 8, 12, 24, 48, 72, 96, 120, 144, 168, 192, 240, 336, 504, 672, 840 |
| 2 | Herceptin Hylecta | ICR, Female | Subcutaneous injection | 6mg/100IU rHuPH20 | |
| 3 | Herceptin/BMI2004 | ICR, Female | Subcutaneous injection | 6mg/100IU BMI2004 | |

**TABLE 22**

| Group | Sample | Add | Administration method | AUC₍ₗₐₛₜ₎ | AUC_{(inf)} | Tₘₐₓ | Cₘₐₓ | t_{1/2}(hr) |
|---|---|---|---|---|---|---|---|---|
| 1 | Herceptin | - | Subcutaneous injection | 55545.47 | 59593.32 | 72.00 | 198.08 | 220.93 |
| 2 | Herceptin Hylecta | - | Subcutaneous injection | 54907.12 | 55127.11 | 24.00 | 282.99 | 127.07 |
| 3 | Herceptin | BMI2004 | Subcutaneous injection | 61894.85 | 62453.67 | 24.00 | 294.69 | 142.41 |

## Claims

1. A composition for subcutaneous injection, comprising
a hyaluronidase polypeptide in which the selected number of amino acids from an integer of 1 to 203 are consecutively deleted from a C-terminus of a polypeptide consisting of the amino acid sequence of SEQ ID NO: 1; and a drug.

2. The composition for subcutaneous injection of claim 1, wherein the polypeptide is one in which the selected number of amino acids from an integer of 34 to 170 are consecutively deleted from the C-terminus of the amino acid sequence of SEQ ID NO: 1.

3. The composition for subcutaneous injection of claim 1, wherein the polypeptide is one in which the selected number of amino acids from an integer of 34 to 68 are consecutively deleted from the C-terminus of the amino acid sequence of SEQ ID NO: 1.

4. The composition for subcutaneous injection of claim 1, wherein the polypeptide is one in which 34, 68, 102, 136, or 170 amino acids are consecutively deleted from the C-terminus of the amino acid sequence of SEQ ID NO: 1.

5. The composition for subcutaneous injection of claim 1, wherein the polypeptide is one in which a first amino acid is further deleted from the N-terminus of the amino acid sequence of SEQ ID NO: 1.

6. The composition for subcutaneous injection of claim 1, wherein the polypeptide consists of an amino acid sequence of SEQ ID NO: 2, SEQ ID NO: 3, SEQ ID NO: 4, SEQ ID NO: 5, or SEQ ID NO: 6.

7. The composition for subcutaneous injection of claim 1, wherein the polypeptide is glycosylated.

8. The composition for subcutaneous injection of claim 1, wherein the polypeptide is stable at pH 3 to 10.

9. The composition for subcutaneous injection of claim 1, wherein the polypeptide is stable at a temperature of -20 to 45°C.

10. The composition for subcutaneous injection of claim 1, wherein the polypeptide has an enzymatic activity of 57% or higher of an initial activity after storage at pH condition in the range between higher than pH 3 and pH 10 or less for 4 weeks.

11. The composition for subcutaneous injection of claim 1, wherein the polypeptide has an enzymatic activity of 32% or higher of an initial activity after storage at pH condition in the range between pH 3 or higher and less than pH 5 for 4 weeks.

12. The composition for subcutaneous injection of claim 1, wherein the polypeptide has an enzymatic activity of 63% or higher of an initial activity thereof after storage at a temperature less than 0°C for 4 weeks.

13. The composition for subcutaneous injection of claim 1, wherein the polypeptide has an enzymatic activity of 83% or higher of an initial activity thereof after storage at a temperature of 0 to 40°C for 4 weeks.

14. The composition for subcutaneous injection of claim 1, wherein the polypeptide has an enzymatic activity of 52% or higher of an initial activity thereof after storage at a temperature of 40°C or more for 4 weeks.

15. The composition for subcutaneous injection of claim 1, wherein the polypeptide has an activity in the range between higher than one-fold and three-folds or less of that of the polypeptide consisting of an amino acid sequence of SEQ ID NO: 1.

16. The composition for subcutaneous injection of claim 1, wherein the polypeptide has an activity of 120,000 to 150,000 IU/mg.

17. The composition for subcutaneous injection of claim 1, wherein the drug is a protein medicine, antibody, an aptamer, mRNA, siRNA (small interfering RNA), shRNA (short hairpin RNA), miRNA (micro RNA), aiRNA (asymmetric interfering RNA), RNAi (RNA interference), antisense oligonucleotide, or small molecule.

18. The composition for subcutaneous injection of claim 17, wherein the drug is soluble receptor, or a soluble receptor and Fc fusion protein.

19. The composition for subcutaneous injection of claim 1, wherein the drug is an anti-cancer agent, anti-diabetic agent, anti-inflammatory agent, anti-viral agent, or immunomodulatory agent.

20. The composition for subcutaneous injection of claim 1, further comprising a stabilizing agent.

21. The composition for subcutaneous injection of claim 20, wherein the stabilizing agent comprises one or more selected from the group consisting of buffer, a nonionic surfactant, a chelating agent, alkaline metal salt, and alkaline earth metal salt.

22. The composition for subcutaneous injection of claim 21, wherein the buffer is one or more selected from the group consisting of succinate buffer solution, acetate buffer solution, phosphate buffer solution, citrate buffer solution, malonate buffer solution, MES (2-(N-morpholino)ethanesulphonic acid) buffer solution, Tris buffer solution, and glycine buffer solution.

23. The composition for subcutaneous injection of claim 21, wherein the nonionic surfactant is polyoxyethylene-sorbitan fatty acid ester.

24. The composition for subcutaneous injection of claim 23, wherein the nonionic surfactant is one or more selected from the group consisting of Polysorbate 20, Polysorbate 80, and Triton X-100.

25. The composition for subcutaneous injection of claim 21, wherein the chelating agent is ethylenediaminetetraacetic acid (EDTA).

26. The composition for subcutaneous injection of claim 21, wherein the alkaline earth metal salt is MgCl₂ and/or MgSO₄.

27. A pharmaceutical composition for treating cancer, comprising a hyaluronidase polypeptide in which the selected number of amino acids from an integer of 1 to 203 are consecutively deleted from a C-terminus of a polypeptide consisting of the amino acid sequence of SEQ ID NO: 1; and an anti-cancer agent.
